(19) Europäisches Patentamt
European Patent Office
Office européen des brevets

(11) EP 3 725 298 A1

(12) EUROPEAN PATENT APPLICATION

(43) Date of publication:
21.10.2020 Bulletin 2020/43

(21) Application number: 19382298.8

(22) Date of filing: 16.04.2019

(51) Int Cl.:
*A61K 9/00* (2006.01) *A61K 9/08* (2006.01)
*A61K 31/454* (2006.01) *A61K 31/58* (2006.01)
*A61K 47/10* (2017.01) *A61K 47/12* (2006.01)
*A61K 47/18* (2017.01) *A61K 47/24* (2006.01)
*A61K 47/38* (2006.01) *A61K 47/40* (2006.01)
*A61K 47/69* (2017.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FAES FARMA, S.A.**
**48940 Leioa - Vizcaya (ES)**

(72) Inventors:
- **HERNÁNDEZ HERRERO, Gonzalo**
  **E-48940 Leioa, Vizcaya (ES)**
- **GONZALO GOROSTIZA, Ana**
  **E-48940 Leioa, Vizcaya (ES)**
- **MARCIANES MORENO, Patricia**
  **E-48940 Leioa, Vizcaya (ES)**
- **GONZÁLEZ FERNÁNDEZ, Paula**
  **E-48940 Leioa, Vizcaya (ES)**
- **TATO CERDEIRAS, Paloma**
  **E-48940 Leioa, Vizcaya (ES)**

(74) Representative: **ABG Intellectual Property Law, S.L.**
**Avenida de Burgos, 16D**
**Edificio Euromor**
**28036 Madrid (ES)**

# (54) STABLE AND PRESERVED PHARMACEUTICAL COMPOSITIONS OF BILASTINE

(57) The invention relates to an aqueous pharmaceutical composition comprising:
a) bilastine, or a pharmaceutically acceptable salt or solvate thereof,
b) benzalkonium chloride, in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,
c) a suspending agent, and
d) 2-hydroxypropyl-β-cyclodextrin;
wherein the pH of the aqueous pharmaceutical composition is between 3.5 and 5.5.

The invention also relates to said compositions for use in the treatment and/or prevention of a disorder or disease susceptible to amelioration by antagonism of $H_1$ histamine receptor and/or of a corticosteroid-responsive disease through nasal administration.

The invention also relates to a process for preparing the aqueous pharmaceutical composition above mentioned.

EP 3 725 298 A1

## Description

### FIELD OF THE INVENTION

The present invention relates to aqueous pharmaceutical compositions of bilastine and, optionally, mometasone or a pharmaceutically acceptable derivative thereof, and to a method for preparing said aqueous pharmaceutical compositions. The present invention also relates to said compositions for use in the treatment and/or prevention of a disorder or disease susceptible to amelioration by antagonism of $H_1$ histamine receptor and optionally, of a corticosteroid-responsive disease, wherein said aqueous pharmaceutical composition is intranasally administered.

### BACKGROUND

It has long been known that histamine plays a very important role in allergic-type diseases, such as allergic rhinitis, conjunctivitis, rhinoconjunctivitis, dermatitis, urticaria and asthma. Antihistaminic compounds acting at the Hi-receptor histamine level are useful for treating such conditions. In this sense, documents EP 0818454 A1 and EP 0580541 A1 as well as patent application EP14382576.8 disclose benzimidazole compounds with selective $H_1$ antihistaminic activity and devoid of arrhythmogenic effects.

A particular compound with the above properties is 2-[4-(2-{4-[1-(2-ethoxyethyl)-1H-benzimidazol-2-yl]-1-piperidinyl}ethyl)phenyl]-2-methylpropanoic acid, also known as bilastine, having formula:

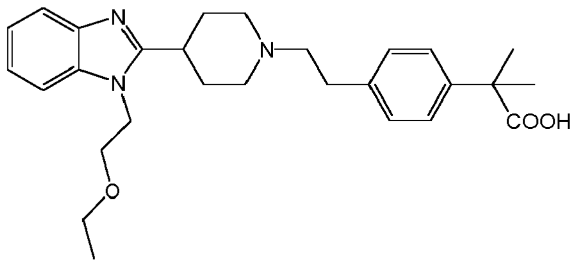

and developed by Faes Farma, Spain. Bilastine is a $H_1$ antagonist benzimidazole compound with no sedative side effects, no cardiotoxic effects, and no hepatic metabolism. In addition, bilastine has proved to be effective for the symptomatic treatment of allergic rhinoconjunctivitis and urticaria.

If a pharmaceutical composition does not itself have adequate antimicrobial activity, the European Pharmacopoeia (Ph. Eur.) recommends adding antimicrobial preservatives, particularly to aqueous preparations, to prevent proliferation which, during normal conditions of storage and use, present a hazard to the patient due to infection and spoilage of the pharmaceutical composition. The efficacy of an antimicrobial preservative may be enhanced or diminished by the active constituent of the pharmaceutical composition. The Ph. Eur. recommends challenging the pharmaceutical composition with a prescribed inoculum of suitable microorganisms, selected from at least the following four: *Pseudomonas aeruginosa* (*P. aeruginosa*) ATCC 9027 (bacteria, Gram negative); *Staphylococcus aureus* (*S. aureus*) ATCC 6538 (bacteria, Gram positive); *Candida albicans* (*C. albicans*) ATCC 10231 (yeast) and *Aspergillus brasiliensis* (*A. brasiliensis*) ATCC 16404 (mould).

*Burkholderia cepacia* (*B. cepacia*) is a gram-negative, nonfermenting bacillus that is found in liquid reservoirs, moist environments, plant surfaces, and soil. In 2003, an outbreak of *B. cepacia* complex among pediatric patients in a Hospital in Denver, Colorado was reported. The epidemiologic investigation led to the identification of the intrinsic contamination of a nasal spray with *B. cepacia* and resulted in a voluntary product recall by the manufacturer [Susan A. Dolan et al. An Outbreak of Burkholderia cepacia Complex Associated with Intrinsically Contaminated Nasal Spray. Infect Control Hosp Epidemiol 2011 ;32(8):804-810)]. *B. cepacia* is a common contaminant of an increasing variety of nonsterile medical products. Despite the fact that this microorganism poses little medical risk to healthy individuals, *B. cepacia* in a nasal spray could cause upper airway colonization and secondarily lead to respiratory infections in individuals with a compromised immune system or those with chronic lung conditions, such as cystic fibrosis. In addition, *B. cepacia* is resistant to many antibiotics and may be difficult to eradicate in this sensitive population if an infection occurs. Thus, additional measures should be considered to assure the safety of nasal products that may be used in high-risk patients.

WO 2005/082416 discloses that attempts at using preservatives in pharmaceutical compositions comprising cyclodextrins only led to satisfactory results for a reduced number of preservatives. Particularly, WO 2005/082416 discloses that compositions with benzalkonium chloride did not comply with USP/Ph. Eur. requirements. WO 2005/082416 is silent regarding combining benzalkonium chloride with other preservatives.

WO 2012/094283 discloses increasing benzalkonium chloride concentration from 0.005% to 0.0125% to achieve

a formulation that successful passes the USP 51 Preservative Efficacy Test for *A. brasiliensis* (*niger*), *C. albicans, E. coli, S. aureus* and *P. aeruginosa.*

Conversely, the inventors have surprisingly found out that for the particular case of a pharmaceutical composition comprising bilastine, β-cyclodextrin and at least one suspending agent, the preservative benzalkonium chloride alone did not provide adequate antimicrobial activity against at least one of the tested micro-organisms.

WO 2013/078500 discloses the use of from 1 to 30 % w/v of a co-solvent in order to maintain the efficacy of the preservatives selected from m-cresol, chlorocresol, methylparaben, ethylparaben, propylparaben, butylparaben, boric acid, phenol, phenylethanol, phenoxyethanol, and mixtures thereof in pharmaceutical compositions suitable for injection. The examples show an amount of at least 10% of co-solvent is used. However, this document is silent regarding the possibility of achieving antimicrobial efficacy without the use of said co-solvent. Furthermore, no compositions for nasal administration are disclosed.

On the other hand, steroids, particularly corticosteroids, are believed to be helpful in alleviating respiratory disorders. In particular, glucocorticoids are believed to block many of the inflammatory pathways activated in respiratory disorders. Moreover, corticosteroids reduce or prevent inflammation of the airways contributing to the treatment of asthma symptoms, chronic obstructive pulmonary disease, or treating inflammation of the nasal passages in allergic conditions such as hay fever. The glucocorticoids for respiratory disorders such as asthma are preferably administered by inhalation to reduce the incidence of steroid-related side effects linked to systemic delivery.

A therapeutic composition has been recently reported in CN103784462 that combines the antihistaminic effect of bilastine with the anti-inflammatory effect of steroids for intranasal or eye drops. However, the low solubility of the bilastine in water impedes the proper administration of the disclosed pharmaceutical compositions via nasal.

KR 2013 0030606 A and KR 2010 0119632 relate to pharmaceutical compositions comprising an antihistamine (which is not bilastine) and mometasone. US 2006/045850 A1 relates to the use of a cyclodextrin in improving the solubility of a steroid in an anti-inflammatory composition. EP 1 894 559 A1 addresses the problem of solubilizing poorly soluble corticoids for aerosol therapy by adding a cyclodextrin. US 2007/082870 A1 addresses the problem of increasing the aqueous solubility of an antifungal azole using cyclodextrins.

Conversely, the inventors have surprisingly found out that for the particular case of a pharmaceutical composition comprising mometasone, the preservative benzalkonium chloride alone was already sufficient to provide adequate antimicrobial activity against micro-organisms. This result was not observed when bilastine was present.

None of the cited documents refer to stable compositions comprising both bilastine and mometasone, which pass the Ph. Eur. standards of efficacy of antimicrobial preservation.

Therefore, there is a need in the art for a nasal pharmaceutical composition with antihistaminic activity having adequate antimicrobial activity. The addition of a corticosteroid further improves the composition, which can then be used for simultaneously treating corticosteroid-responsive diseases of the airway passage and/or lungs.

## BRIEF DESCRIPTION OF THE INVENTION

The present invention provides aqueous pharmaceutical compositions comprising bilastine that have antimicrobial activity against at least four of the six microorganisms *Pseudomonas aeruginosa, Staphylococcus aureus, Candida albicans, Aspergillus brasiliensis, Escherichia coli* and *Burkholderia cepacia.* This is achieved by including at least two preservatives in the composition, selected from a non-arbitrary group of preservatives.

Thus, in a first aspect the invention relates to an aqueous pharmaceutical composition comprising:

a) bilastine or a pharmaceutically acceptable salt or solvate thereof,

b) benzalkonium chloride, in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,

c) a suspending agent,

d) 2-hydroxypropyl-β-cyclodextrin;

wherein the pH of the aqueous pharmaceutical composition is between 3.5 and 5.5.

The inventors have surprisingly found that by selecting a specific combination of benzalkonium chloride and a further preservative, the resulting pharmaceutical composition passes the Ph. Eur. standards for antimicrobial efficacy.

In a second aspect the invention relates to a process for preparing an aqueous pharmaceutical composition comprising:

a) preparing an aqueous solution comprising

a1. 2-hydroxypropyl-β-cyclodextrin,

a2. benzalkonium chloride in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,

a3. bilastine or a pharmaceutically acceptable salt or solvate thereof,

a4. a buffer agent to obtain an aqueous solution having a pH of between 3.5 and 5.5,

b) preparing an aqueous suspension of a suspending agent,

c) preparing a mixture by adding the aqueous solution of step a) to the aqueous suspension of step b) and,

d) homogenising the mixture of the preceding step under stirring, optionally adding a further buffer to reach a pH of between 3.5 and 5.5.

In a third aspect the invention relates to an aqueous pharmaceutical composition as defined above for use as a medicament.

Another aspect of this invention refers to an aqueous pharmaceutical composition as defined above for use in the treatment and/or prevention of a disorder or disease susceptible to amelioration by antagonism of $H_1$ histamine receptor and/or of a corticosteroid-responsive disease.

In another aspect the invention relates to a nasal spray device comprising the aqueous pharmaceutical composition above described.

These aspects and preferred embodiments thereof are additionally also defined in the claims.

## BRIEF DESCRIPTION OF THE FIGURES

Figure 1A shows the change in the solubility of bilastine with the content of 2-hydroxypropyl-β-cyclodextrin in the range of pH of between 4.3 and 4.9.

Figure 1B shows the change in the solubility of mometasone furoate with the content of 2-hydroxypropyl-β-cyclodextrin.

Figure 2 shows the change in the solubility of bilastine with the content of α-cyclodextrin in a pH of 4.5.

Figure 3A shows the influence of kolliphor RH40 in the solubility of bilastine.

Figure 3B shows the influence of kolliphor RH40 in the solubility of mometasone furoate monohydrate.

Figure 4A shows that there is a linear behaviour between the percentage of dissolved mometasone and the percentage of mometasone impurities.

Figure 4B shows that the HPBCD cyclodextrin minimizes the solubilisation of mometasone when used at a concentration lower than 85 mg/mL.

## DETAILED DESCRIPTION OF THE INVENTION

The present invention provides aqueous pharmaceutical compositions containing bilastine and, optionally, mometasone or a pharmaceutically acceptable derivative thereof.

Besides, the stability and homogeneity of the aqueous pharmaceutical composition of the invention allows its effective administration by nasal spray.

In a first aspect, the invention relates to an aqueous pharmaceutical composition comprising:

a) bilastine or a pharmaceutically acceptable salt or solvate thereof,

b) benzalkonium chloride, in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,

c) a suspending agent,

d) 2-hydroxypropyl-β-cyclodextrin;

wherein the pH of the aqueous pharmaceutical composition is between 3.5 and 5.5.

In a preferred embodiment, the aqueous pharmaceutical composition of the invention comprises:

a) bilastine or a pharmaceutically acceptable salt or solvate thereof,

b) benzalkonium chloride, in combination with at least one further preservative selected from the group consisting of phenylethyl alcohol, benzyl alcohol, sodium benzoate, chlorbutanol, phenoxyethanol, cetrimide and 2-(ethylmercuriomercapto)benzoic acid sodium salt,

c) a suspending agent,

d) 2-hydroxypropyl-β-cyclodextrin;

wherein the pH of the aqueous pharmaceutical composition is between 3.5 and 5.5.

In a further preferred embodiment, the aqueous pharmaceutical composition of the invention comprises:

a) bilastine or a pharmaceutically acceptable salt or solvate thereof,

b) benzalkonium chloride, in combination with at least one further preservative selected from the group consisting of phenylethyl alcohol, benzyl alcohol, cetrimide and 2-(ethylmercuriomercapto)benzoic acid sodium salt,

c) a suspending agent,

d) 2-hydroxypropyl-β-cyclodextrin;

wherein the pH of the aqueous pharmaceutical composition is between 3.5 and 5.5.

The inventors have surprisingly found that by selecting a specific combination of benzalkonium chloride and a further preservative, the resulting pharmaceutical composition passes the Ph. Eur. standards for antimicrobial efficacy. The Ph. Eur. recommends challenging the pharmaceutical composition with a prescribed inoculum of suitable microorganisms, selected from at least the following four: *Pseudomonas aeruginosa* (*P. aeruginosa*) ATCC 9027 (bacteria, Gram negative); *Staphylococcus aureus* (*S. aureus*) ATCC 6538 (bacteria, Gram positive); *Candida albicans* (*C. albicans*) ATCC 10231 (yeast) and *Aspergillus brasiliensis* (*A. brasiliensis*) ATCC 16404 (mould).

Advantageously, the present invention provides pharmaceutical compositions of bilastine for nasal administration, with antimicrobial efficacy against these four microorganisms and preferably also against at least one of the two microorganisms *Escherichia coli* (*E. coli*) ATCC 8739 (bacteria, Gram negative) and *Burkholderia cepacia* (*B. cepacia*) ATCC 17759 (bacteria, Gram negative).

"Pharmaceutical composition" as used herein, relates to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The expression "aqueous pharmaceutical composition" refers to a liquid pharmaceutical composition comprising water. In the context of the present invention, the term "aqueous" means that said composition comprises water, preferable at least 1 wt% of water with respect to the total weight of the composition, more preferably at least 10 wt% of water, more preferably at least 20 wt% of water, more preferably at least 30 wt% of water, more preferably at least 40 wt% of water, more preferably at least 50 wt% of water, more preferably at least 60 wt% of water, more preferably at least 70 wt% of water, more preferably at least 80 wt% of water, more preferably at least 85 wt% of water, more preferably at least 90 wt% of water. In a particularly preferred embodiment, the aqueous pharmaceutical compositions of the present

invention comprise at least 80 wt% of water with respect to the total weight of the composition.

The present invention further provides aqueous pharmaceutical compositions containing bilastine and optionally mometasone or a pharmaceutically acceptable derivative thereof. In particular, the combination of bilastine with mometasone or a pharmaceutically acceptable derivative thereof allows the treatment and/or prevention of disorders or diseases susceptible to amelioration by antagonism of $H_1$ histamine receptor, and of corticosteroid-responsive diseases though nasal administration.

The inventors of the present invention have surprisingly found that a content of less than 8.5 wt% of 2-hydroxypropyl-β-cyclodextrin (HPBCD) in the aqueous pharmaceutical composition of the invention improves the solubility of bilastine, and solubilises a minimum quantity of the steroid mometasone or pharmaceutically acceptable derivative thereof, avoiding the degradation of the steroid mometasone, thereby maintaining the homogeneity and stability of the aqueous pharmaceutical composition.

In the context of the present disclosure, the terms "wt%", or "wt%", refer to the same meaning, mass fraction (expressed as percentage by mass). The mass fraction of a substance within a mixture is the ratio of the mass of that substance to the total mass of the mixture expressed, with a denominator of 100, as percentage by mass.

In a preferred embodiment, the aqueous pharmaceutical composition of the invention is characterized by further comprising:

e) mometasone or a pharmaceutically acceptable derivative thereof selected from an ester, ether and ketonide derivative, and wherein the content of 2-hydroxypropyl-β-cyclodextrin is less than 8.5 wt%.

The components of the aqueous pharmaceutical composition of the invention are further described below.

*Bilastine*

The aqueous pharmaceutical composition of the invention comprises a compound of formula:

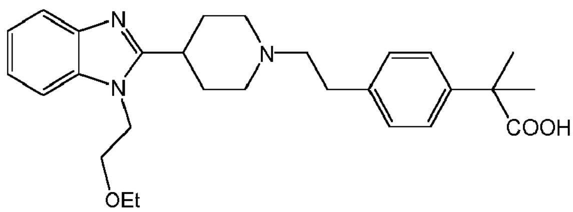

or a pharmaceutically acceptable salt or solvate thereof. This compound is the 2-[4-(2-{4-[1-(2-ethoxyethyl)-1H-benzimidazol-2-yl]-1-piperidinyl}ethyl)phenyl]-2-methylpropanoic acid, also known as bilastine. The synthesis of bilastine has been described in documents EP 0818454 A1 and EP 0580541 A1, and the patent application EP14382576.8.

Bilastine may be in the form of salts or solvates, preferably pharmaceutically acceptable salts or solvates.

The term "pharmaceutically acceptable salts" as used herein encompasses any salt with no limitation on the type of salt that can be used, provided that these are acceptable for administration to a patient, meaning that they do not induce undue toxicity, irritation, allergic responses, or the like. Pharmaceutically acceptable salts are well known in the art. By way of illustration, pharmaceutically acceptable salts of bilastine can be acid addition salts, base addition salts or metal salts, and can be synthesized from the parent compounds containing a basic or acid moiety by means of conventional chemical processes known by the persons skilled in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, camphorsulfonate, etc. Illustrative examples of base addition salts include inorganic base salts such as, for example, ammonium salts and organic base salts such as, for example, ethylenediamine, ethanolamine, *N,N*-dialkylenethanolamine, triethanolamine, glutamine, amino acid basic salts, etc. Illustrative examples of metal salts include, for example, sodium, potassium, calcium, magnesium, aluminum and lithium salts.

The term "solvate" according to this invention is to be understood as meaning any form of the active compound according to the invention which has another molecule (most likely a polar solvent) attached to it via non-covalent bonding. Examples of solvates include hydrates and alcoholates. Solvation methods are generally known in the state of the art.

The bilastine in the aqueous pharmaceutical composition of the invention is also meant to include compounds which differ only in the presence of one or more isotopically enriched atoms. For example, compounds having the present

structures except for the replacement of a hydrogen by a deuterium or tritium, or the replacement of a carbon by a $^{13}C$- or $^{14}C$-enriched carbon or a nitrogen by $^{15}N$-enriched nitrogen are within the scope of this invention.

In a particular embodiment, the amount of bilastine in the aqueous pharmaceutical composition of the invention is equal to, or greater than, 0.1, 0.2, 0.3, 0.4, 0.5, 0.6, 0.7 or 0.8 wt%, based on the total weight of the composition.

In another particular embodiment, the amount of bilastine in the aqueous pharmaceutical composition of the invention is equal to, or lower than, 1.0, 0.9, 0.8, 0.7, 0.6, 0.5, 0.4 or 0.3 wt%, based on the total weight of the composition.

In a preferred embodiment, the amount of bilastine in the aqueous pharmaceutical composition of the invention is comprised between 0.2 and 0.8 wt%, based on the total weight of the composition. Preferably, the amount of bilastine in the aqueous pharmaceutical composition of the present invention is comprised between 0.3 and 0.7 wt%, more preferably between 0.4 and 0.6 wt%. In a more preferred embodiment, the amount of bilastine in the aqueous pharmaceutical composition of the invention is 0.4 wt%.

Mometasone

In a particular embodiment, the aqueous pharmaceutical composition of the invention may further contain mometasone and/or a pharmaceutically acceptable derivative thereof. Generally, steroids, such as mometasone, possess regulatory functions in cells, tissues and organisms.

The term "pharmaceutically acceptable derivatives thereof" refers to non-toxic functional equivalents or derivatives of mometasone, which can be obtained by substitution of atoms or molecular groups or bonds of mometasone, whereby the basic structure is not changed, and which differ from the structure of mometasone in at least one position. Particularly, pharmaceutically acceptable derivatives of mometasone in the context of the present invention refer to an ester, ether or ketonide of mometasone. That is, to compounds wherein at least one of the hydroxyl groups of mometasone is functionalized as an ester, ether or ketonide. Thus, in a particular embodiment the aqueous pharmaceutical composition of the invention comprises a pharmaceutically acceptable derivative of mometasone selected from ester, ether and ketonide.

In a particular embodiment, an ester derivative of mometasone refers to mometasone wherein at least one -OH group is replaced by a -OC(O)R' group, wherein R' is selected from $C_1$-$C_6$ alkyl, halo($C_1$-$C_6$ alkyl), ($C_6$-$C_{12}$)aryl($C_1$-$C_6$)alkyl, $C_3$-$C_7$ cycloalkyl, 3- to 10-membered heterocyclyl, $C_6$-$C_{12}$ aryl, and 3- to 10-membered heteroaryl. In a particular embodiment, R' is selected from $C_1$-$C_6$ alkyl, and 3- to 10-membered heteroaryl, such as methyl, ethyl, propyl, butyl, furyl, thiophenyl or pyridinyl. In a particular embodiment, the ester derivative is a furoate or a propionate, such as mometasone furoate and mometasone propionate. In a preferred embodiment, the aqueous pharmaceutical composition of the present invention contains mometasone furoate.

In a particular embodiment, an ether derivative of mometasone refers to mometasone wherein at least one -OH group is replaced by a -OR' group, wherein R' is selected from $C_1$-$C_6$ alkyl, halo($C_1$-$C_6$ alkyl), ($C_6$-$C_{12}$)aryl($C_1$-$C_6$)alkyl, $C_3$-$C_7$ cycloalkyl, 3- to 10-membered heterocyclyl, $C_6$-$C_{12}$ aryl, and 3- to 10-membered heteroaryl.

In a particular embodiment, a ketonide derivative of mometasone refers to mometasone wherein two -OH groups disposed either on contiguous carbons or on two carbons having one carbon disposed between them, taken together form a group of formula

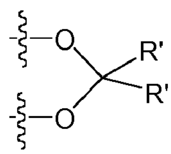

,

wherein each R' is independently selected from $C_1$-$C_6$ alkyl, halo($C_1$-$C_6$ alkyl), ($C_6$-$C_{12}$)aryl($C_1$-$C_6$)alkyl, $C_3$-$C_7$ cycloalkyl, 3- to 10-membered heterocyclyl, $C_6$-$C_{12}$ aryl, and 3- to 10-membered heteroaryl. In a particular embodiment, each R' is independently selected from $C_1$-$C_6$ alkyl, such as methyl, ethyl, propyl or butyl. In a particular embodiment, the ketonide derivative is an acetonide, i.e. R' is methyl, such as triamcinolone acetonide.

Methods for the preparation of these mometasone derivatives are well-known in the art (e.g. e.g. M.B. Smith, J. March, March's Advanced Organic Chemistry, Wiley-Interscience, 5th ed.). Likewise, the mometasone and mometasone derivatives may be present in the aqueous pharmaceutical composition both as free compounds or as solvates (e.g., hydrates, alcoholates, etc.), both forms being included within the scope of the present invention. Thus, for example, suitable forms of mometasone furoate in the aqueous pharmaceutical composition of the invention include anhydrous form or hydrate form, such as monohydrate form. In a preferred embodiment, the aqueous pharmaceutical composition of the invention contains mometasone hydrate. The solvation methods are well known in the state of the art.

In a particular embodiment, the amount of mometasone or a pharmaceutically acceptable derivative thereof in the aqueous pharmaceutical composition of the invention is equal to, or greater than, 0.01, 0.02, 0.03, 0.04, 0.05, or

0.06 wt% based on the total weight of the composition.

In another particular embodiment, the amount of mometasone or a pharmaceutically acceptable derivative thereof in the aqueous pharmaceutical composition of the invention is equal to, or lower than, 0.08, 0.07, 0.06, 0.05, 0.04, or 0.03 wt% based on the total weight of the composition.

In a preferred embodiment, the amount of mometasone or a pharmaceutically acceptable derivative thereof in the aqueous pharmaceutical composition of the invention is comprised between 0.02 and 0.06 wt% based on the total weight of the composition, preferably 0.05 wt%.

Preservative

In a particular embodiment, the pharmaceutical composition of the invention comprises at least two preservatives selected from the group comprising benzalkonium chloride, phenylethyl alcohol, benzyl alcohol, sodium benzoate, chlorbutanol, phenoxyethanol, cetrimide and 2-(ethylmercuriomercapto)benzoic acid sodium salt (also known as thimerosal).

In a preferred embodiment, the pharmaceutical composition of the invention comprises the preservative benzalkonium chloride in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt.

In a further preferred embodiment, the pharmaceutical composition of the invention comprises the preservative benzalkonium chloride in combination with at least one further preservative selected from the group consisting of phenylethyl alcohol, benzyl alcohol, sodium benzoate, chlorbutanol, phenoxyethanol, cetrimide and 2-(ethylmercuriomercapto)benzoic acid sodium salt; more preferably at least one further preservative selected from the group consisting of phenylethyl alcohol, benzyl alcohol, cetrimide and 2-(ethylmercuriomercapto)benzoic acid sodium salt.

In the context of the present application, the terms "preservative efficacy", "preservative effectiveness" or "antimicrobial efficacy" mean that the composition satisfies Ph. Eur. standards for nasal preparations (European Pharmacopoeia 9.0 - 5.1.3. Efficacy of Antimicrobial Preservation - 01/2014:50104). According to Ph. Eur. standards, the recommended efficacy to be achieved in the product examined is determined by criteria A. In justified cases where the A criteria cannot be attained, the B criteria must be satisfied, if a preservative is to be deemed as effective. The acceptance criteria, in terms of decrease in the number of microorganisms with time, vary for different types of preparations according to the degree of protection intended. The table below summarizes the criteria for evaluation of antimicrobial activity in terms of the $\log_{10}$ reduction in the number of viable microorganisms against the value obtained for the inoculum, and can be found in the cited Ph. Eur. chapter 5.1.3.

Table 1. Criteria of preservative acceptance in Nasal preparations according to Ph. Eur.

| | $Log_{10}$ reduction | | | | |
|---|---|---|---|---|---|
| | criteria | 2d | 7d | 14d | 28d |
| Bacteria | A | 2 | 3 | - | NI |
| | B | - | - | 3 | NI |
| Fungi | A | - | - | 2 | NI |
| | B | - | - | 1 | NI |

NI: no increase in the number of viable microorganisms compared to the previous reading.

Preservatives can be used to inhibit microbial growth (e.g., bacterial or fungi) in the compositions. An "effective amount" of a preservative is that amount necessary to prevent the growth of microorganisms in the composition within criteria A or B.

In a particular embodiment of the present invention, the minimum period during which the pharmaceutical composition of the invention is effectively preserved is 7 days or more, preferably 14 days or more, more preferably 28 days or more.

The present invention allows the pharmaceutical compositions to be stored in a container, wherein the composition is effectively preserved for a period of at least 7 days, preferably 14 days or more, more preferably 28 days or more starting from the day the container is opened.

In a more preferred embodiment, the preservative is able to prevent microbial growth in the composition for a storage period of at least six months.

The Ph. Eur. recommends challenging the pharmaceutical composition with a prescribed inoculum of suitable microorganisms, selected from at least the following four: *Pseudomonas aeruginosa* (*P. aeruginosa*) ATCC 9027 (bacteria, Gram negative); *Staphylococcus aureus* (*S. aureus*) ATCC 6538 (bacteria, Gram positive); *Candida albicans* (*C.*

*albicans*) ATCC 10231 (yeast) and *Aspergillus brasiliensis* (*A. brasiliensis*) ATCC 16404 (mould).

The present invention has the advantage that it provides nasal pharmaceutical compositions of bilastine with antimicrobial efficacy against these four microorganisms but also preferably against at least one of the two microorganisms *Escherichia coli* (*E. coli*) ATCC 8739 (bacteria, Gram negative) and *Burkholderia cepacia* (*B. cepacia*) ATCC 17759 (bacteria, Gram negative) in the challenge tests.

In a particular embodiment, the pharmaceutical composition of the invention complies with the Ph. Eur. standards for antimicrobial efficacy against *P. aeruginosa, S. aureus, Candida albicans* and *A. brasiliensis.* Preferably, the pharmaceutical composition of the invention complies with the Ph. Eur. standards for antimicrobial efficacy against *P. aeruginosa, S. aureus, C.albicans, A. brasiliensis* and at least one of the two microorganisms selected from *B. cepacia* and *E. coli.* More preferably, the pharmaceutical composition of the invention complies with the Ph. Eur. standards for antimicrobial efficacy against *P. aeruginosa, S. aureus, C.albicans, A. brasiliensis* and *B. cepacia.* Even more preferably, the pharmaceutical composition of the invention complies with the Ph. Eur. standards for antimicrobial efficacy against *P. aeruginosa, S. aureus, C.albicans, A. brasiliensis, B. cepacia* and *E. coli.*

As described herein, the inventors have surprisingly found out that a pharmaceutical composition comprising bilastine, β-cyclodextrin, at least one suspending agent and the preservative benzalkonium chloride alone did not have adequate antimicrobial activity against at least one of the six tested micro-organisms. Furthermore, the surprising finding extended to realizing that the pharmaceutical composition of the invention was not sufficiently preserved when using any given combination of more than one preservative, even when including benzalkonium chloride as one of the preservatives.

Therefore, in a preferred embodiment of the present invention, the at least two preservatives of the pharmaceutical composition are selected from a group comprising benzalkonium chloride, phenylethyl alcohol, benzyl alcohol, sodium benzoate, chlorbutanol, phenoxyethanol, cetrimide and 2-(ethylmercuriomercapto)benzoic acid sodium salt. 2-(Ethylmercuriomercapto)benzoic acid sodium salt is also known as thimerosal.

In a preferred embodiment, the at least two preservatives of the pharmaceutical composition are selected from a group comprising phenylethyl alcohol, thimerosal, cetrimide, benzyl alcohol and benzalkonium chloride.

Preferably, one of the at least two preservatives is benzalkonium chloride.

In a preferred embodiment compatible with all embodiments described herein, the at least two preservatives of the pharmaceutical composition have a synergistic effect in the antimicrobial efficacy of the resulting composition against at least one of the microorganisms selected from the group consisting of *P. aeruginosa, S. aureus, C. albicans, A. brasiliensis, E. coli* and *B. cepacia.*

In a preferred embodiment, compatible with any other embodiment disclosed herein, the at least two preservatives present in the pharmaceutical composition of the invention are not selected from the group consisting of EDTA, potassium sorbate in combination with EDTA, p-chlorocresol, chlorhexidine, methylparaben in combination with propylparaben.

In a preferred embodiment, the pharmaceutical composition of the invention comprises at least 0.0005 wt%, 0.001 wt%, 0.002 wt%, 0.005 wt%, 0.01 wt%, 0.05 wt%, 0.1 wt%, 0.15 wt% or 0.2 wt% of a combination of preservatives.

In another preferred embodiment, which can be compatible with the former, the pharmaceutical composition of the invention comprises no more than 0.2 wt%, 0.3 wt%, 0.4 wt%, 0.5 wt%, 0.6 wt%, 0.7 wt%, 0.8 wt%, 0.9 wt%, 1 wt%, 1.1 wt%, 1.2 wt%, 1.3 wt%, 1.4 wt% or 1.5 wt% of a combination of preservatives.

Preferred embodiments of the invention are those where the combination of preservatives is in an amount comprised between 0.001 wt% and 1.5 wt%. Preferably, between 0.001 wt% and 1.1 wt%, between 0.005 wt% and 1.1 wt%, between 0.01 wt% and 1.1 wt%, more preferably between 0.01 wt% and 1 wt%, most preferably between 0.02 wt% and 1 wt%.

In a preferred embodiment, the pharmaceutical composition of the present invention does not comprise a co-solvent.

In another preferred embodiment, the preservative in the pharmaceutical composition of the present invention is not first dissolved in a co-solvent and then added to the bulk solution.

In a most preferred embodiment, the at least two preservatives in the pharmaceutical composition of the present invention are added to water.

In the context of the present invention, said co-solvent is a compound that aids in solubilizing the preservative, wherein the mixture of preservative and co-solvent is prepared separately by dissolving the preservative in the co-solvent and this is later added to the bulk solution. The co-solvent as defined herein is selected from ethanol, glycerin, propylene glycol, isopropyl alcohol, glycerol formal, tetraglycol, polyethylene glycol and mixtures thereof in an amount comprised between 1 and 30 % w/v.

## Suspending agent

The term "suspending agent" refers to an agent that overcomes agglomeration of dispersed particles in a liquid

medium, and increases the viscosity of the medium so that the particles settle more slowly (Remington, The Science and practice of pharmacy, 21st edition, p.1072, 2005).

In a particular embodiment, the suspending agent in the aqueous pharmaceutical composition of the invention is selected from cellulose and/or cellulose derivatives wherein the hydroxyl groups of cellulose have been partially or fully substituted to provide cellulose ethers (-OR). In a preferred embodiment, the suspending agent in the aqueous pharmaceutical composition of the invention is a cellulose ether derivative selected from microcrystalline cellulose (MCC), methyl cellulose, carboxymethyl cellulose, sodium carboxymethylcellulose (Na-CMC), hydroxypropyl-methyl cellulose (HPMC) or mixtures thereof. Suspending agents suitable for the pharmaceutical composition of the present invention are commercially available, for example, under the trade name Vivapur® MCG 811P (JRS Pharma), Avicel® RC591 (FMC Biopolymer) and Avicel® RC581 (JRS Pharma).

Particularly, when the aqueous pharmaceutical composition of the invention is applied in nasal delivery devices, Avicel RC591 and Vivapur MCG are preferably used due to its thixotropic properties. Both compounds form a gel network that keeps drug particles suspended in nasal delivery devices. During agitation and pumping, the gel becomes fluid and enables an eased spraying that results in an efficient, standardized and optimum atomization and deposition pattern. After agitation, the fluid regains its viscosity preventing the dripping from the nose or outflow into the throat area, prolonging the retention time of the drug substance in the nasal cavity. Preferably, Avicel RC-591 is used at 20 mg/mL (2.0 wt%), while a concentration of 18 mg/mL (1.8 wt%) of Vivapur MCG 811P is recommended in the aqueous pharmaceutical composition of the invention.

The aqueous pharmaceutical composition of the present invention contains between 1.0 and 2.5 wt% of the suspending agent based on the total weight of the composition, preferably between 1.3 and 2 wt%, even more preferably between 1.6 and 1.9 wt%, even more preferably 1.8 wt%. The inventors have found that a content lower than 1 wt% produces too fluid dispersions, while a content over this value produces thixotropic gels.

<u>2-hydroxypropyl-β-cyclodextrin</u>

Bilastine is only slightly soluble in water, and its solubility is pH-dependent, becoming more hydrophilic at pH<3.6 and pH>8.5 and more hydrophobic at pH in the range of 3.8-8.5. Therefore, in the range of pH of the aqueous pharmaceutical composition of the invention of between 3.5 and 5.5, a solubiliser of bilastine is required.

The aqueous pharmaceutical composition of the present invention therefore also comprises 2-hydroxypropyl-3-cyclodextrin (HPBCD).

In addition, HPBCD in the pharmaceutical composition of the invention also masks the unpleasant flavours of the composition, favouring the administration of the pharmaceutical composition to the patient.

In the context of the present invention HPBCD may have different degrees of substitution. For example, Cavasol W7 HP™ and Cavasol W7 HP5™ (Ashland) having a degree of substitution of between 4.1 and 5.1, Cavitron W7 HP7™(Ashland), Kleptose HPB™ (Roquette's) having a degree of substitution of 4.5, Kleptose HP™ (Roquette's) having a degree of substitution of 5.6 (HP8BCD) and Trappsol having a degree of substitution of 3.5 and 6.5 (CTD) are commercially available.

When the pharmaceutical composition of the invention further comprises mometasone, the inventors have surprisingly found that that there is a direct linear relationship between the percentages of solubilized mometasone and the resulting impurities. In addition, the inventors found that the presence of HPBCD in the present pharmaceutical composition allows the provision of a formulation comprising bilastine and mometasone which is stable in the sense that it minimizes the degradation of the steroid below pharmaceutical acceptable levels while it ensures that the proper amount of bilastine is solubilised. In this sense, while HPBCD produces the solubilisation of bilastine in the range of pH of the aqueous pharmaceutical composition of the invention, other cyclodextrines, such as α-cyclodextrine (a-CD), do not lead to the required solubility. Figure 1A shows the change of the solubility of bilastine with the content of hydroxypropyl-β-cyclodextrin in a pH range of between 4.3 and 4.9. In particular, figure 1A shows that for a content of 25 mg/mL of hydroxypropyl-β-cyclodextrin (2.5 wt%) the solubility of bilastine is around 10 mg/mL at a pH value of 4.6. By contrast, when 5 wt% of α-cyclodextrine (a-CD) is used instead of HPBCD, the solubility of bilastine does not reach 1 mg/mL, as figure 2 shows at a pH of 4.5.

Moreover, figure 1B shows that the solubility of mometasone furoate remains around 4 mg/mL when the content of HPBCD is 75 mg/mL (7.5 wt%). In this sense, the inventors have also found that a content of HPBCD of less than 8.5 wt% in the aqueous pharmaceutical composition of the invention, produces the complete dissolution of bilastine while, at the same time, a minimum quantity of mometasone is dissolved, thereby minimizing the unwanted degradation of the steroid below pharmaceutical acceptable levels.

In a preferred embodiment, the pharmaceutical composition is such that content of 2-hydroxypropyl-β-cyclodextrin is less than 8.5 wt%.

As described throughout the text, and unless stated otherwise, a content of less than 8.5 wt% is to be understood as a content of less than 85 mg/mL of formulation. This is because 1 mL of the pharmaceutical composition weights

approximately 1000 mg.

In a particular embodiment, the aqueous pharmaceutical composition of the present invention contains at least 1, 2, 3, 4, 5, 6, 7, 8 wt% of HPBCD. In another particular embodiment, the aqueous pharmaceutical composition of the present invention contains no more than 9, 8, 7, 6, 5, 4, 3 wt% of HPBCD.

In a particular embodiment, the aqueous pharmaceutical composition of the present invention contains between 1 and 8.5 wt% of HPBCD. In another particular embodiment, the aqueous pharmaceutical composition of the present invention contains between 2 and 8.5 wt% of HPBCD.

In yet another particular embodiment, the aqueous pharmaceutical composition of the present invention contains less than 5 wt% of HPBCD.

In a particular embodiment the aqueous pharmaceutical composition of the present invention contains between 1 and 5 wt% of HPBCD. In a preferred embodiment the content of HPBCD is comprised between 2 and 4 wt%, more preferably the content of HPBCD is 3 wt%.

## pH

The pH of the aqueous pharmaceutical composition of the invention is between 3.5 and 5.5.

In a particular embodiment, the pH of the aqueous pharmaceutical composition of the invention is comprised between 4.0 and 5.0. In another particular embodiment the pH of the aqueous pharmaceutical composition is between 4.3 and 4.9

In a preferred embodiment the pH of the aqueous pharmaceutical composition of the invention is 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4 or 5.5. Preferably, the pH of the aqueous pharmaceutical composition of the invention is 4.0, 4.1, 4.2, 4.3, 4.4, 4.5, 4.6, 4.7, 4.8, 4.9 or 5.0. More preferably, the pH of the aqueous pharmaceutical composition of the invention is 4.3, 4.4, 4.5, 4.6, 4.7, 4.8 or 4.9.

The aqueous pharmaceutical composition of the invention has been developed for nasal administration. The physiological pH of the nasal cavity, particularly of the human nasal cavity, is between about 5.5-6.5, and increases in rhinitis to about 7.2-8.3.

In a preferred embodiment, the aqueous pharmaceutical composition further comprises a buffer agent. As used herein, the term "buffer agent" refers to an agent that imparts suitable pH characteristics to the aqueous pharmaceutical composition provided herein. Said buffer agents are used for adjusting the pH of the compositions of the invention to a pH of from 3.5 to 5.5, more preferably to a pH of from 4.0 to 5.0, even more preferably to a pH of between 4.3 to 4.9, even more preferably 4.6.

The pH values mentioned in the present application have been measured at room temperature with a pHmeter, particularly by direct reading from pHmeter Crison Microph 2000. In the context of the present invention, the pH of the pharmaceutical composition of the invention may be measured by any other device suitable for measuring the pH in the composition.

In a particular embodiment, the aqueous pharmaceutical composition of the invention comprises a buffering agent selected from acetate buffer, citrate buffer, phosphate buffer, borate buffer, or a combination thereof. In a preferred embodiment, the buffer agent is selected from sodium citrate hemihydrates, citric acid anhydrous and mixtures thereof. More preferably, the buffer agent is selected from citric acid monohydrate and trisodium citrate dihydrate.

In a particular embodiment, the aqueous pharmaceutical composition contains between 0.15 and 0.20 wt% of a buffer agent. In a preferred embodiment, the aqueous pharmaceutical composition contains between 0.17 and 0.19 wt% of a buffer agent. More preferably, the aqueous pharmaceutical composition of the invention contains 2.0 mg/mL of citric acid monohydrate.

The pH range of the aqueous pharmaceutical composition of the invention maintains the chemical, physical, and/or physiological stability of the aqueous pharmaceutical composition and is well-tolerated by the nasal cavities.

## Additional excipients

The aqueous pharmaceutical composition of the invention may further comprise additional excipients. In a particular embodiment the aqueous pharmaceutical composition of the invention comprises a humectant, a surfactant, a tonicity agent, and/or combinations thereof.

Preferably, the aqueous pharmaceutical composition comprises between 0.005% and 0.03 wt% of a surfactant, and between 1 and 3 wt% of a humectant.

In another particular embodiment, the aqueous pharmaceutical composition of the invention further comprises a humectant selected from anhydrous glycerine, glycerol and propylene glycol. Preferably, the humectant in the aqueous pharmaceutical composition of the invention is anhydrous glycerine.

In another particular embodiment, the aqueous pharmaceutical composition of the invention contains a surfactant agent. It is believed that the surfactant agent may lower the surface tension of the composition, easing the

manufacturing process. Examples of suitable surfactant agents may be selected from, but not limited to polyethoxylated sorbitan derivatives such as polysorbates, their ether ethoxylates, produced by reaction of sorbitan esters with ethylene oxide, polyoxyethylene alkyl phenol, polyoxyethylene cetyl ether, polyoxyethylene alkyl-aryl ether, polyoxyethylene monolaurate, polyoxyethylene vegetable oil, polyoxyethylene sorbitan monolaurate, polyoxyethylene esters or mixed fatty and resin acids, polyoxyethylene sorbitol lanolin derivative, polyoxyethylene tridecylether, polyoxyethylene sorbitan esters of mixed fatty and resin acids, polyethoxylated sorbitan derivatives or esters of fatty acids (e.g. Polysorbates), polyoxyethylene sorbitan monostearate, polyoxyethylene sorbitan monooleate, polyoxyethylene monostearate, polyoxyethylene stearyl ether, polyoxyethylene oleyl ether, polyoxyethylene tridecyl ether, polyoxyethylene fatty alcohol, polyoxyethylene alkyl amine, polyoxyethylene glycol monopalmitate, polyoxyethylene sorbitan monopalmitate, polyoxyethylene cetyl ether, polyoxyethylene oxypropylene stearate, polyoxyethylene lauryl ether, polyoxyethylene lanolin derivative, sodium oleate, quaternary ammonium derivative, potassium oleate, N-cetyl N-ethyl morpholinium ethosulfate, sodium lauryl sulfate or mixtures thereof. Particularly preferred surfactants are Polysorbate 80, Polysorbate 40, Polysorbate 60, sorbitan monolaurate, sorbitan monopalmitate, sorbitan monostearate, polyoxyl 40 hydrogenated castor oil (Cremophor RH 40), polyoxyethylene alkyl ether (-BO-10V, Cremophor A20, Cremophor A25), poloxamers, phospholipids and propylene glycol. In a preferred embodiment, the aqueous pharmaceutical composition of the invention comprises between 0.005 and 0.03 wt% of surfactant. Preferably, the aqueous pharmaceutical composition of the invention comprises Polysorbate 80 as surfactant agent. Polysorbate 80 may lower the surface tension of the composition, easing the manufacturing process. In a preferred embodiment, the aqueous pharmaceutical composition of the invention comprises between 0.005 and 0.03 wt% of Polysorbate 80.

In another particular embodiment, the aqueous pharmaceutical composition comprises a tonicity agent. In particular, when the aqueous pharmaceutical composition of the invention is used as nasal spray suspension, the tonicity agent may help to maintain the osmolarity of the aqueous pharmaceutical composition as close as possible to the physiological values. In a preferred embodiment the tonicity agent in the aqueous pharmaceutical composition of the invention is anhydrous glycerine, sorbitol, mannitol or propylene glycol. In a more preferred embodiment the tonicity agent is anhydrous glycerine. More preferably, the aqueous pharmaceutical composition of the invention contains 21 mg/mL (2.1 wt%) of anhydrous glycerine.

<u>Process for preparing the aqueous pharmaceutical composition of the invention</u>

In one aspect the invention relates to the process for preparing the aqueous pharmaceutical composition of the invention.

The process of the invention comprises:

a) preparing an aqueous solution comprising

  a1. 2-hydroxypropyl-β-cyclodextrin,

  a2. benzalkonium chloride in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,

  a3. bilastine or a pharmaceutically acceptable salt or solvate thereof,

  a4. a buffer agent to obtain an aqueous solution having a pH of between 3.5 and 5.5,

b) preparing an aqueous suspension of a suspending agent,

c) preparing a mixture by adding the aqueous solution of step a) to the aqueous suspension of step b) and,

d) homogenising the mixture of the preceding step under stirring, optionally adding a further buffer to reach a pH of between 3.5 and 5.5.

The process of the invention leads to the preparation of the aqueous pharmaceutical composition. Therefore, all particular and preferred embodiments disclosed above for the pharmaceutical composition, especially those referring to the type of component and amounts used, also apply to the process of the invention.

According to step a) of the process of the invention, the order in which the components are added is not important.

The inventors have found that the presence of bilastine or a pharmaceutically acceptable salt or solvate thereof increases the pH of the aqueous solution, thereby a buffer agent is usually necessary to maintain the pH between 3.5

and 5.5.

Preferably, the buffer agent is firstly added to water, followed by the addition of the cyclodextrin and then the bilastine.

The preservatives may be added as the first reagents, before the addition of the buffer agent, or only after the addition of the bilastine. In this way, in a particular embodiment, the preservatives are the first component to be added in the process of the invention. In another particular embodiment, the preservatives are only added to the aqueous solution once bilastine is dissolved.

Also, optionally, the components can be added to the aqueous solution under stirring conditions.

In a preferred embodiment, a buffer agent is added to the aqueous solution in any moment of the process to reach a pH of between 3.5 and 5.5, preferably between 4 and 5, more preferably between 4.3 and 4.9, even more preferably between 4.4 and 4.6, even more preferably 4.45. Preferably, the buffer agent is selected from citric acid monohydrate or trisodium citrate dihydrate.

The pH values mentioned in the present application were measured with a pHmeter, particularly by direct reading from pHmeter Crison Microph 2000. The buffer agent is preferably added to the aqueous solution under stirring conditions.

In one particular embodiment, a buffer agent is added to the aqueous solution in steps a) and/or d).

In a preferred embodiment, the amounts of bilastine, HPBCD and preservatives in the aqueous pharmaceutical composition of the invention are as described above for the composition of the invention.

According to step b) of the process of the invention, an aqueous suspension of a suspending agent is prepared. In a particular embodiment the suspending agent is as described above for the pharmaceutical composition. In another particular embodiment, it is in the amounts described above for the pharmaceutical composition.

The aqueous suspension of step b) can be prepared by dissolving the suspending agent in deionized water under stirring conditions. The aqueous suspension can be further homogenized by stirring. Preferably, the aqueous suspension is homogenized by stirring to obtain a suspension phase. The homogeneity assessment is carried out by observing the sample to detect the absence of phase separation or agglomerates.

Particularly, microcrystalline cellulose (MCC) and/or sodium carboxymethylcellulose (Na-CMC) requires an activation step to work as suspending agent in the aqueous pharmaceutical composition of the invention. In a particular embodiment, the suspending agent selected from microcrystalline cellulose (MCC), sodium carboxymethylcellulose (Na-CMC) and mixtures thereof are activated by dispersion in water and applying high shear forces. The high shear forces broke up the particles and allow the formation of the gel network.

In a preferred embodiment the suspending agent is activated by firstly preparing an aqueous solution of the suspending agent containing a volume of water comprised between 30% and 40% with respect to the total volume, preferably between 33% and 37%, even more preferably between 34% and 36% volume of water, even more preferably 35% volume of water. The suspending agent is added to water under stirring. The resulting aqueous suspension is homogenised by applying high shear forces. The homogeneity assessment is carried out by observing the sample to detect the absence of phase separation or agglomerates.

In a particular embodiment, a tonicity agent is further added in the process of the invention.

More preferably, the tonicity agent is added either in step a) or in step b) of the process of the invention. Particularly, when the aqueous pharmaceutical composition of the invention is used as nasal spray suspension, the tonicity agent helps to maintain the osmolarity of the aqueous pharmaceutical composition as close as possible to the physiological values. Preferably, the tonicity agent in the aqueous pharmaceutical composition of the invention is anhydrous glycerine or glycerol, more preferably anhydrous glycerine. More preferably, the aqueous pharmaceutical composition of the invention contains 21 mg/mL (2.1 wt%) of anhydrous glycerine.

Moreover, optionally, humectants can be added to the aqueous suspension of the suspending agent under stirring conditions. In one particular embodiment, in step b) an aqueous suspension of a suspending agent further containing a humectant is prepared. In another preferred embodiment, in step b) an aqueous suspension of a suspending agent, further containing a humectant, and a tonicity agent is prepared.

In a particular embodiment, the process for preparing an aqueous pharmaceutical composition of the invention further comprises a step of adding mometasone.

Preferably, the process comprises a further step of:

b1) preparing a dispersion of mometasone, or a pharmaceutically acceptable derivative thereof selected from an ester, ether and ketonide derivative with a surfactant in purified water,

wherein the dispersion of step b1) is added to the mixture of step c), before the homogenizing step d), and wherein the content of 2-hydroxypropyl-3-cyclodextrin in step a) is less than 8.5 wt%.

The inventors have found that a content of HPBCD of less than 8.5 wt% in the aqueous pharmaceutical composition of the invention produces the complete dissolution of bilastine while, at the same time, a minimum quantity of mometasone is dissolved, thereby preventing the unwanted degradation of the steroid below pharmaceutical acceptable levels.

According to step b1), a dispersion is formed by dispersing mometasone or a pharmaceutically acceptable derivative thereof with a surfactant in purified water. Surfactants lower the surface tension of the dispersion increasing the homogeneity and stability. Moreover, the surfactant eases the wetting and dispersion of mometasone or a pharmaceutically acceptable derivative thereof in water, thereby facilitating the manufacturing process. Suitable surfactants in this step include Polysorbate 80. In a preferred embodiment a dispersion is formed by dispersing mometasone or a pharmaceutically acceptable derivative thereof together with between 0.005 and 0.03 wt% of Polysorbate 80.

The mometasone and mometasone derivatives to be dispersed in step b1) may be in free form or as solvates (e.g., hydrates, alcoholates, etc.), both forms being included within the scope of the present invention. The solvation methods are generally known in the state of the art. Preferably, the solvate is a hydrate.

In a preferred embodiment, a dispersion is formed by dispersing mometasone furoate with a surfactant in purified water. More preferably, mometasone furoate is in anhydrous form or in a hydrate form, such as monohydrate form.

In a particular embodiment, the amount of mometasone or a pharmaceutically acceptable derivative in the dispersion is comprised between 0.02 and 0.06 wt% based on the total weight of the composition, preferably 0.05 wt%.

In step c) of the process of the invention, the aqueous solution of step a) is added to the aqueous suspension of step b), and optionally, the dispersion of mometasone from step b1) is also added. The resulting mixture is homogenised by stirring in step d).

Optionally, a buffer agent can be added to the homogenized mixture containing the steroid to obtain the final formulation with a pH of between 3.5 and 5.5. In one preferred embodiment, the pH of the final formulation is of between 4.0 and 5.0, more preferably of between 4.3 and 4.9. In a more preferred embodiment the pH of the final formulation is about 4.6. Suitable buffering agents include acetate buffer, a citrate buffer, a phosphate buffer, a borate buffer, or a combination thereof. Preferably, the buffer agent is selected from sodium citrate hemihydrates, citric acid anhydrous and mixtures thereof.

## Uses

Bilastine has been found to be an antagonist of histamine $H_1$ receptor and would thus be useful in the treatment and/or prevention of diseases known to be susceptible to improvement by antagonism of histamine $H_1$ receptor. The skilled person readily identifies which diseases are known to be susceptible to improvement by antagonism of histamine $H_1$ receptor. As an example, such diseases are allergic rhinitis, allergic conjunctivitis, urticaria, CNS diseases (Simons, F. Estelle R., and Keith J. Simons. "Histamine and H1-antihistamines: celebrating a century of progress." Journal of Allergy and Clinical Immunology 128.6 (2011): 1139-1150) or redness, itching and swelling, rhinorrhea, bronchoconstriction, anaphylaxis, urticaria as well as regulation of food intake and sleep, convulsion, and attention (Kalpaklioglu, Fusun, and Ayse Baccioglu. "Efficacy and safety of H1-antihistamines: an update." Anti-Inflammatory & Anti-Allergy Agents in Medicinal Chemistry (Formerly Current Medicinal Chemistry-Anti-Inflammatory and Anti-Allergy Agents) 11.3 (2012): 230-237).

Moreover, mometasone or pharmaceutically acceptable derivatives thereof in the aqueous pharmaceutical composition of the invention reduces or prevents inflammation of the airways passages contributing to alleviate respiratory disorders.

Therefore, an aspect of the invention refers to the aqueous pharmaceutical composition of the invention for use as a medicament.

Another aspect of the invention refers to an aqueous pharmaceutical composition of the invention for use in the treatment and/or prevention of a disorder or disease susceptible to amelioration by antagonism of $H_1$ histamine receptor and/or of a corticosteroid-responsive disease. A preferred embodiment refers to the aqueous pharmaceutical composition of the invention for use in the treatment and/or prevention of a disorder or disease susceptible to amelioration by antagonism of $H_1$ histamine receptor and/or of a corticosteroid-responsive disease, wherein said aqueous pharmaceutical composition is intranasally administered.

The invention also refers to the method for the treatment and/or prevention of a disorder or disease susceptible to amelioration by antagonism of $H_1$ histamine receptor and/or a corticosteroid-responsive disease comprising administering an effective amount of an aqueous pharmaceutical composition of the invention. A preferred embodiment refers to the method for the treatment and/or prevention of a disorder or disease susceptible to amelioration by antagonism of $H_1$ histamine receptor and/or a corticosteroid-responsive disease comprising administering an effective amount of an aqueous pharmaceutical composition of the invention, wherein said aqueous pharmaceutical composition is intranasally administered.

The invention also refers to the use of the aqueous pharmaceutical composition of the invention for the manufacture of a medicament for the treatment and/or prevention of a disorder or disease susceptible to amelioration by antagonism of $H_1$ histamine receptor and/or a corticosteroid-responsive disease. A preferred embodiment, refers to the use of the aqueous pharmaceutical composition of the invention for the manufacture of a medicament for the treatment and/or prevention of a disorder or disease susceptible to amelioration by antagonism of $H_1$ histamine receptor and/or a

corticosteroid-responsive disease, wherein said aqueous pharmaceutical composition is intranasally administered.

The invention provides an aqueous pharmaceutical composition for use in the treatment and/or prevention of a disorder or disease susceptible to amelioration by antagonism of $H_1$ histamine receptor. Thus, in a particular embodiment the invention relates to an aqueous pharmaceutical composition for use in the treatment and/or prevention of a disorder or disease susceptible to amelioration by antagonism of $H_1$ histamine receptor wherein said disorder or disease is selected from rhinitis, conjunctivitis and rhinoconjunctivitis. Preferably, the aqueous pharmaceutical composition is intranasally administered.

The invention also provides an aqueous pharmaceutical composition for use in the treatment of a corticosteroid-responsive disease. In a particular embodiment, the invention also provides an aqueous pharmaceutical composition for use in the treatment of a corticosteroid-responsive disease selected from asthma, allergic and non-allergic rhinitis, non-malignant proliferative and inflammatory diseases. Preferably, the aqueous pharmaceutical composition is intranasally administered.

The term "treatment" or "to treat" in the context of this specification means administration of the aqueous pharmaceutical composition according to the invention to ameliorate or eliminate the disease or one or more symptoms associated with said disease. "Treatment" also encompasses ameliorating or eliminating the physiological sequelae of the disease.

The term "ameliorate" in the context of this invention is understood as meaning any improvement on the situation of the patient treated.

The term "prevention" or "to prevent" in the context of this specification means administration of a compound or formulation according to the invention to reduce the risk of acquiring or developing the disease or one or more symptoms associated with said disease.

Pharmaceutical forms

Topical administration of the aqueous pharmaceutical composition of the invention to the nasal cavities may be accomplished utilizing nasal spray devices such as a metered-dose spray pump or single- and duo-dose spray devices. Therefore, an aspect of the invention is directed to a nasal spray device comprising the aqueous pharmaceutical composition of the invention. In a preferred embodiment, the nasal spray comprising the aqueous pharmaceutical composition of the invention, is a metered-dose spray pump.

Solutions may be administered intranasally by inserting an appropriate device (such as a nasal spray bottle and actuator) into each nostril. Active drug is then expelled from the nasal spray device.

## EXAMPLES

### Materials and methods

Materials

The compounds and microorganisms used in the following examples can be easily procured in usual providers. In the present case, as an example, Bilastine was provided by Neuland Laboratories limited, Mometasone furoate monohydrate was provided by Sterling, hydroxypropyl-β-cyclodextrin was provided by Roquette, Polysorbate 80 by Seppic, MCC and Na-CMC by JRS Pharma (Vivapur® MCG) or FMC (Avicel RC 591), Citric Acid monohydrate by Brenntag, anhydrous glycerine was supplied by KLK Oleo, benzalkonium chloride and Trisodium citrate dihydrate were both supplied by Merck. Preservatives were obtained from the indicated providers: phenylethyl alcohol (Sigma-Aldrich-Merck), thimerosal (Sigma-Aldrich-Merck), cetrimide (Guinama), benzyl alcohol (Merck), EDTA (Brentag), potassium sorbate (Celanese), p-chlorocresol (Panreac), chlorhexidine (Guinama), methylparaben and propylparaben (Clariant Produckte GmbH).

The water used in the following examples was purified water obtained using an Elix® water purification system from Merck-Millipore.

Microorganisms *Pseudomonas aeruginosa* (*P. aeruginosa*) ATCC 9027 (bacteria, Gram negative), *Staphylococcus aureus* (*S. aureus*) ATCC 6538 (bacteria, Gram positive), *Candida albicans* (*C. albicans*) ATCC 10231 (yeast), *Aspergillus brasiliensis* (*A. brasiliensis*) ATCC 16404 (mould), *Escherichia coli* (*E. coli*) ATCC 8739 (bacteria, Gram negative) and *Burkholderia cepacia* (*B. cepacia*) ATCC 17759 (bacteria, Gram negative) were purchased from CECT (Colección Española de Cultivos Tipo, Valencia, Spain).

The microorganisms used herein were not obtained as a result of access to samples of Brazilian Genetic Heritage or Associated Traditional Knowledge. Also, no biological material used in the invention has been obtained, procured or maintained from India.

HPLC measurements were conducted using a HPLC-HCLASS Chromatographic System with PDA or UV-VIS

detector equipped with a Xbridge Shield RP18 3.5μm 4.6μm x 250mm Column. Mobile phases were ammonium bicarbonate (10 mM pH 9, FLUKA), acetonitrile (FISCHER) and methanol (SCHARLAU). Samples were submitted to Acrodisc 32mm Sryinge Filters with 1.2 μm Supor Membrane, Batch: 18-1077 (PALL).

Representative manufacture technique of the formulation

Representative aqueous pharmaceutical compositions belonging to the invention can be prepared following at least one of the two processes explained below. The skilled person readily understands that if a formulation of the invention comprising a different excipient or a different amount of a component is to be prepared, it suffices to adapt the following process to the desired final composition.

Process I - Bilastine nasal formulation (no further API)

Stage I: preparation of a representative aqueous solution of bilastine

The present example is not to be considered a limitation of the invention and, among others, the order of addition of the reagents is not relevant.

0.215 g of benzalkonium chloride (preservative) were added to 430 mL of water and the mixture stirred until the preservative was completely solubilized (5 min of magnetic stirrer at 340 rpm). Subsequently, 5 g of benzyl alcohol were added and the mixture stirred until the second preservative was completely solubilized (5 min of magnetic stirrer at 340 rpm).

Anhydrous glycerin (21 g) was then added to the aqueous solution and the resulting mixture kept stirring for 5 min (magnetic stirrer at 340 rpm). 2 g of citric acid monohydrate were then added to the mixture and the solution was kept stirring for 5 min (magnetic stirrer at 340 rpm).

Then, 2-hydroxypropyl-3-cyclodextrin (6.25 g) was added to the aqueous solution and the mixture was stirred to obtain a homogenous solution (58 min of magnetic stirrer at 340 rpm). 2 g of Bilastine were added and the solution was kept stirring for further 45 min (magnetic stirrer at 340 rpm).

While stirring, a buffer agent (trisodium citrate dehydrate, 1.94 g) was added to reach a pH of 4.45 in the aqueous solution of bilastine.

Stage II: preparation of an aqueous suspension of a suspending agent

An aqueous suspension of a suspending agent was prepared by adding 18 g of Avicel RC 591 (microcrystalline cellulose (MCC) and sodium carboxymethylcellulose (Na-CMC)) to 400 mL of purified water under stirring (at 1100 rpm for 35 min). The resulting aqueous suspension is then homogenized with high shear forces (at 22000 rpm for 5 min). The resulting aqueous suspension is left to rest for about 15 min.

Stage III: Combining the solution phase and the suspension phase.

The solution phase of bilastine obtained in stage I above was added to the suspension phase and homogenized with stirring for 10 min (900 rpm). Purified water (120.2 g) was added to that the total volume was 1L, while maintaining the stirring conditions for 10 further minutes (800 rpm).

The pH of the composition was measured with pHmeter Crison Microph 2000 at room temperature, and 0.37 g of trisodium citrate dehydrate were added to reach a pH of 4.6.

The homogeneity assessment of the Bilastine nasal formulation I was done by observing the sample and confirming the absence of phase separation or presence of agglomerates.

Process II - Bilastine and mometasone nasal formulation

Stage I: preparation of a representative aqueous solution of bilastine

An aqueous solution of bilastine and mometasone can be prepared by following the process described below.

Citric acid monohydrate was added to purified water to reach a pH of 4.45 and the solution was stirred to obtain a uniform and homogenous solution. Then, 2-hydroxypropyl-β-cyclodextrin (25 mg/mL) was added to the aqueous solution and the mixture was stirred to obtain a homogenous solution.

(2-[4-(2-{4-[1-(2-ethoxyethyl)-1H-benzimidazol-2-yl]-1-piperidinyl}ethyl)phenyl]-2-methylpropanoic acid) or bilastine was then added and the solution was stirred to obtain a homogenous solution. Benzalkonium chloride (preservative) was subsequently added to the aqueous solution of bilastine maintaining the stirring conditions. Further, a buffer

agent (trisodium citrate dehydrate) was added maintaining the stirring conditions to reach a pH of 4.45 in the aqueous solution of bilastine.

Stage II: preparation of a dispersion of mometasone

Polysorbate 80 was added to purified water under stirring. Then, Mometasone furoate monohydrate was added to the solution of Polysorbate 80 maintaining the stirring conditions for about 20 min.

Stage III: preparation of an aqueous suspension of a suspending agent

An aqueous suspension of a suspending agent is prepared by adding Vivapur MCG 811P to purified water under stirring. The resulting aqueous suspension is then homogenized with high shear forces. The resulting aqueous suspension is left to rest for about 15 min.

Anhydrous glycerine is added to the aqueous suspension while stirring.

Stage IV: combining the solution phase and the suspension phase to obtain the Final representative formulation II.

The solution phase of bilastine was added to the suspension phase and homogenized by stirring. Then, the dispersion of mometasone furoate was added maintaining the conditions of stirring for around further 10 min. Purified water was added up to the total weight maintaining the conditions of stirring .

The pH of the composition was measured with pHmeter Crison Microph 2000 at room temperature, and when necessary trisodium citrate dehydrate was added to reach a pH of 4.6.

The homogeneity assessment of the Bilastine and mometasone nasal formulation II was done by observing the sample and confirming the absence of phase separation or presence of agglomerates.

Example 1 - Bilastine nasal formulations of the invention

The formulations shown in this example were prepared following Process I described above, where the further preservative was added simultaneously with the benzalkonium chloride (BAK). The formulations comprised the following components:

| Component | mg/mL |
|---|---|
| Bilastine | 8 |
| Hydroxypropyl-β-cyclodextrin | 25 |
| Citric acid monohydrate | 3 |
| Trisodium citrate dihydrate | q.s pH 4.6 |
| Anhydrous glycerine | 21 |
| Microcrystalline cellulose and sodium carboxymethylcellulose (Avicel RC 591) | 18 |
| benzalkonium chloride (BAK) | 0.2 |
| Further preservative(s) | See table below |
| Purified water | q.s. 1 mL |

In addition, each formulation varied in the further preservative type and/or amount as shown in the table below.

| Formulation | Further preservative | mg/mL |
|---|---|---|
| FF181077 | Chlorhexidine | 0.02 |
| FF181078 | Chlorhexidine | 0.06 |
| FF181079 | Cetrimide | 1 |
| FF181080 | Cetrimide | 10 |
| FF181081 | Phenylethanol | 2.5 |

(continued)

| Formulation | Further preservative | mg/mL |
|---|---|---|
| FF181082 | Phenylethanol | 5 |
| FF181083 | Thimerosal | 0.02 |
| FF181084 | Thimerosal | 0.05 |
| FF181085 | Chlorocresol | 0.05 |
| FF181086 | Methyl-paraben | 0.33 |
| | Propyl-paraben | 0.17 |
| FF180430 | EDTA | 1 |
| FF180431 | EDTA | 3 |
| FF180432 | EDTA | 5 |
| FF180433 | Benzyl alcohol | 5 |
| FF180434 | Potassium sorbate | 0.08 |
| | EDTA | 5 |

**Example 2 - Protocol for preparing the contaminated samples**

In order to evaluate the preservative efficacy of the formulations, Ph. Eur. 9th edition standards were followed, which consist in the artificial contamination of the formulations with at least four of the six microbes in the table below at a microbe concentration comprised between $10^5$ and $10^6$ CFU/mL (colony-forming units per milliliter).

| Microorganism | |
|---|---|
| *Pseudomonas aeruginosa (P. aeruginosa)* | ATCC 9027 (bacteria, Gram negative) |
| *Staphylococcus aureus (S. aureus)* | ATCC 6538 (bacteria, Gram positive) |
| *Candida albicans (C. albicans)* | ATCC 10231 (yeast) |
| *Aspergillus brasiliensis (A. brasiliensis)* | ATCC 16404 (mould) |
| *Escherichia coli (E. coli)* | ATCC 8739 (bacteria, Gram negative) |
| *Burkholderia cepacia (B. cepacia)* | ATCC 17759 (bacteria, Gram negative) |

The microorganisms, taken from cryogenic Microbank™ vials, were inoculated on Tryptic (trypticase) soy agar (TSA) tubes, except for C. albicans, which was inoculated on Sabouraud Dextrose Agar (SDA) tube. The inoculated tubes were incubated at 30-35 °C for 24 hours except C.albicans, which was inoculated for 48 hours at 20-25 °C.

Once grown, the cultures were diluted in NaCl 9 g/mL to obtain a concentration of $10^8$ CFU/mL.

Each formulation was then contaminated. In general, 17 mL of each formulation was contaminated with no more than 170 μL of inoculum, so that no more than 1% in volume was added. The resulting mixture was homogenized gently. The contaminated formulations were kept at a temperature of 20-25 °C and in the dark throughout the experiment.

A formulation without any contamination was also evaluated, as well as inoculated samples without the components of the pharmaceutical composition of the invention.

The formulations were then sampled at day 2, 7, 14 and 28.

**Example 3 - Antimicrobial efficacy of the formulations of Example 1**

The following tables summarize the results obtained from counting the number of CFU after 0, 2, 7, 14 and 28 days of having prepared the contaminated samples of the formulations of Example 1, following the protocol described in Example 2.

Table 2. Challenge test for FF181079 - BAK (0.02%) and Cetrimide (0.1%)

| Microorganism | Elapsed time (days) | | | | | Criteria |
|---|---|---|---|---|---|---|
| | 0 | 2 | 7 | 14 | 28 | |
| *P. aeruginosa* | $1.0 \times 10^6$ | <10 | <10 | <10 | <10 | **A** |
| *S. aureus* | $1.4 \times 10^6$ | $<10^2$ | $<10^2$ | $<10^2$ | $<10^2$ | **A** |
| *C. albicans* | $1.2 \times 10^6$ | $1.6 \times 10^5$ | $1.1 \times 10^4$ | $1.4 \times 10^3$ | <10 | **A** |
| *A. brasiliensis* | $1.1 \times 10^6$ | $6.5 \times 10^4$ | $4.0 \times 10^4$ | $4.5 \times 10^4$ | $3.0 \times 10^4$ | **B** |
| Results represented as CFU/mL | | | | | | |

Table 3. Challenge test for FF181080 - BAK (0.02%) and Cetrimide (1%)

| Microorganism | Elapsed time (days) | | | | | Criteria |
|---|---|---|---|---|---|---|
| | 0 | 2 | 7 | 14 | 28 | |
| *P. aeruginosa* | $1.0 \times 10^6$ | $<10^2$ | $<10^2$ | $<10^2$ | $<10^2$ | **A** |
| *S. aureus* | $1.4 \times 10^6$ | $<10^3$ | $<10^3$ | $<10^3$ | $<10^3$ | **A** |
| *C. albicans* | $1.2 \times 10^6$ | <10 | <10 | <10 | <10 | **A** |
| *A. brasiliensis* | $1.1 \times 10^6$ | $<10^2$ | $<10^2$ | $<10^2$ | $<10^2$ | **A** |
| Results represented as CFU/mL | | | | | | |

Table 4. Challenge test for FF181081 - BAK (0.02%) and Phenylethanol (0.25%)

| Microorganism | Elapsed time (days) | | | | | Criteria |
|---|---|---|---|---|---|---|
| | 0 | 2 | 7 | 14 | 28 | |
| *P. aeruginosa* | $1.0 \times 10^6$ | <10 | <10 | <10 | <10 | **A** |
| *S. aureus* | $1.4 \times 10^6$ | $<10^2$ | $<10^2$ | $<10^2$ | $<10^2$ | **A** |
| *C. albicans* | $1.2 \times 10^6$ | $8.6 \times 10^4$ | $1.3 \times 10^4$ | $4.0 \times 10^2$ | <10 | **A** |
| *A. brasiliensis* | $1.1 \times 10^6$ | $5.0 \times 10^4$ | $1.0 \times 10^4$ | $8.0 \times 10^3$ | $6.5 \times 10^3$ | **A** |
| Results represented as CFU/mL | | | | | | |

Table 5. Challenge test for FF181082 - BAK (0.02%) and Phenylethanol (0.5%)

| Microorganism | Elapsed time (days) | | | | | Criteria |
|---|---|---|---|---|---|---|
| | 0 | 2 | 7 | 14 | 28 | |
| *P. aeruginosa* | $1.0 \times 10^6$ | <10 | <10 | <10 | <10 | **A** |
| *S. aureus* | $1.4 \times 10^6$ | $<10^2$ | $<10^2$ | $<10^2$ | $<10^2$ | **A** |
| *C. albicans* | $1.2 \times 10^6$ | $3.0 \times 10^5$ | $9.2 \times 10^3$ | 7.0 x 10 | <10 | **A** |
| *A. brasiliensis* | $1.1 \times 10^6$ | $5.5 \times 10^2$ | $6.0 \times 10^3$ | <10 | <10 | **A** |
| *B. cepacia* | $3.4 \times 10^6$ | $2.0 \times 10^4$ | 5.0 x 10 | ---* | ---* | **A** |
| Results represented as CFU/mL<br>* Not measured | | | | | | |

Table 6. Challenge test for FF181083 - BAK (0.02%) and Thimerosal (0.002%)

| Microorganism | Elapsed time (days) | | | | | Criteria |
|---|---|---|---|---|---|---|
| | 0 | 2 | 7 | 14 | 28 | |
| *P. aeruginosa* | $1.2 \times 10^6$ | <10 | <10 | <10 | <10 | **A** |
| *S. aureus* | $1.6 \times 10^6$ | <10 | <10 | <10 | <10 | **A** |
| *C. albicans* | $7.5 \times 10^5$ | $<10^2$ | $<10^2$ | $<10^2$ | $<10^2$ | **A** |
| *A. brasiliensis* | $7.0 \times 10^5$ | $<10^2$ | $<10^2$ | $<10^2$ | $<10^2$ | **A** |
| *B. cepacia* | $3.4 \times 10^6$ | <10 | <10 | ---* | ---* | **A** |
| Results represented as CFU/mL<br>* Not measured | | | | | | |

Table 7. Challenge test for FF181084 - BAK (0.02%) and Thimerosal (0.005%)

| Microorganism | Elapsed time (days) | | | | | Criteria |
|---|---|---|---|---|---|---|
| | 0 | 2 | 7 | 14 | 28 | |
| *P. aeruginosa* | $1.2 \times 10^6$ | <10 | <10 | <10 | <10 | **A** |
| *S. aureus* | $1.6 \times 10^6$ | <10 | <10 | <10 | <10 | **A** |
| *C. albicans* | $7.5 \times 10^5$ | $<10^3$ | $<10^3$ | $<10^3$ | $<10^3$ | **A** |
| *A. brasiliensis* | $7.0 \times 10^5$ | $<10^3$ | $<10^3$ | $<10^3$ | $<10^3$ | **A** |
| *B. cepacia* | $3.4 \times 10^6$ | <10 | <10 | ---* | ---* | **A** |
| Results represented as CFU/mL<br>* Not measured | | | | | | |

Table 8. Challenge test for FF180433 - BAK (0.02%) and Benzyl alcohol (0.5%)

| Microorganism | Elapsed time (days) | | | | | Criteria |
|---|---|---|---|---|---|---|
| | 0 | 2 | 7 | 14 | 28 | |
| *P. aeruginosa* | $2.0 \times 10^6$ | <10 | <10 | <10 | <10 | **A** |
| *S. aureus* | $1.3 \times 10^6$ | $<10^2$ | $<10^2$ | $<10^2$ | $<10^2$ | **A** |
| *C. albicans* | $6.1 \times 10^5$ | $1.4 \times 10^5$ | $7.8 \times 10^3$ | $5.3 \times 10^2$ | <10 | **A** |
| *A. brasiliensis* | $1.4 \times 10^6$ | $2.2 \times 10^2$ | 10 | $2.0 \times 10$ | <10 | **A** |
| *B. cepacia* | $1.0 \times 10^6$ | $9.0 \times 10$ | <10 | <10 | <10 | **A** |
| Results represented as CFU/mL | | | | | | |

Table 9. Challenge test for FF181078 - BAK (0.02%) and Chlorhexidine (0.006%)

| Microorganism | Elapsed time (days) | | | | | Criteria |
|---|---|---|---|---|---|---|
| | 0 | 2 | 7 | 14 | 28 | |
| *P. aeruginosa* | $2.0 \times 10^6$ | <10 | <10 | <10 | <10 | **A** |
| *S. aureus* | $1.7 \times 10^6$ | $<10^2$ | $<10^2$ | $<10^2$ | $<10^2$ | **A** |
| *C. albicans* | $6.8 \times 10^5$ | $3.3 \times 10^4$ | $4.9 \times 10^4$ | $1.2 \times 10^4$ | $2.3 \times 10^3$ | **B** |
| *A. brasiliensis* | $9.5 \times 10^5$ | $9.0 \times 10^3$ | $5.5 \times 10^3$ | <10 | $1.8 \times 10^3$ | **No A or B** |

(continued)

| Microorganism | Elapsed time (days) | | | | | Criteria |
|---|---|---|---|---|---|---|
| | 0 | 2 | 7 | 14 | 28 | |
| *B. cepacia* | 3.4 x $10^6$ | 1.1 x $10^7$ | 1.1 x $10^7$ | ---* | ---* | **No A** |
| Results represented as CFU/mL<br>* Not measured | | | | | | |

Table 10. Challenge test for FF181085 - BAK (0.02%) and Chlorocresol (0.005%)

| Microorganism | Elapsed time (days) | | | | | Criteria |
|---|---|---|---|---|---|---|
| | 0 | 2 | 7 | 4 1 | 28 | |
| *P. aeruginosa* | 1.2 x $10^6$ | <10 | <10 | <10 | <10 | **A** |
| *S. aureus* | 1.6 x $10^6$ | <10 | <10 | <10 | <10 | **A** |
| *C. albicans* | 7.5 x $10^5$ | 7.5 x $10^4$ | 3.1 x $10^4$ | 2.0 x $10^5$ | 2.2 x $10^5$ | **No A or B** |
| *A. brasiliensis* | 7.0 x $10^5$ | 4.0 x $10^4$ | 9.5 x $10^3$ | 9.0 x $10^2$ | 4.2 x $10^2$ | **A** |
| *B. cepacia* | 3.4 x $10^6$ | 1.0 x $10^7$ | 1.1 x $10^7$ | ---* | ---* | **No A** |
| Results represented as CFU/mL<br>* Not measured | | | | | | |

Table 11. Challenge test for FF181086 - BAK (0.02%) and Methyl-paraben (0.033%)+ Propyl-paraben (0.017%)

| Microorganism | Elapsed time (days) | | | | | Criteria |
|---|---|---|---|---|---|---|
| | 0 | 2 | 7 | 14 | 28 | |
| *P. aeruginosa* | 1.2 x $10^6$ | <10 | <10 | <10 | <10 | **A** |
| *S. aureus* | 1.6 x $10^6$ | <$10^2$ | <$10^2$ | <$10^2$ | <$10^2$ | **A** |
| *C. albicans* | 7.5 x $10^5$ | 1.2 x $10^5$ | 1.3 x $10^4$ | 2.0 x $10^2$ | <10 | **A** |
| *A. brasiliensis* | 7.0 x $10^5$ | 3.0 x $10^2$ | 3.0 x 10 | 3.0 x $10^2$ | 1.2 x $10^3$ | **No A or B** |
| *B. cepacia* | 3.4 x $10^6$ | 9.2 x $10^6$ | 1.4 x $10^7$ | ---* | ---* | **No A** |
| Results represented as CFU/mL<br>* Not measured | | | | | | |

Table 12. Challenge test for FF180430 - BAK (0.02%) and EDTA (0.1%)

| Microorganism | Elapsed time (days) | | | | | Criteria |
|---|---|---|---|---|---|---|
| | 0 | 2 | 7 | 14 | 28 | |
| *P. aeruginosa* | 2.0 x $10^6$ | <10 | <10 | <10 | <10 | **A** |
| *S. aureus* | 1.3 x $10^6$ | 5.0 x $10^2$ | <10 | <10 | <10 | **A** |
| *C. albicans* | 6.1 x $10^5$ | 1.4 x $10^4$ | 1.2 x $10^3$ | 10 | <10 | **A** |
| *A. brasiliensis* | 1.4 x $10^6$ | 5.5 x $10^5$ | 5.0 x $10^5$ | 1.6 x $10^5$ | 4.0 x $10^4$ | **No A or B** |
| *B. cepacia* | 1.0 x $10^6$ | 2.4 x $10^5$ | 7.1 x $10^6$ | 1.2 x $10^7$ | 7.7 x $10^6$ | **No A or B** |
| Results represented as CFU/mL | | | | | | |

Table 13. Challenge test for FF180431 - BAK (0.02%) and EDTA (0.3%)

| Microorganism | Elapsed time (days) | | | | | Criteria |
|---|---|---|---|---|---|---|
| | 0 | 2 | 7 | 14 | 28 | |
| *P. aeruginosa* | 2.0 x $10^6$ | <10 | <10 | <10 | <10 | **A** |
| *S. aureus* | 1.3 x $10^6$ | 1.8 x $10^3$ | <$10^2$ | <$10^2$ | <$10^2$ | **A** |
| *C. albicans* | 6.1 x $10^5$ | 4.7 x $10^4$ | 1.0 x $10^3$ | 5.0 x 10 | <10 | **A** |
| *A. brasiliensis* | 1.4 x $10^6$ | 2.3 x $10^5$ | 3.5 x $10^5$ | 2.5 x $10^5$ | 9.5 x $10^4$ | **No A or B** |
| *B. cepacia* | 1.0 x $10^6$ | 1.4 x $10^5$ | 5.4 x $10^4$ | 4.8 x $10^6$ | 9.2 x $10^6$ | **No A or B** |
| Results represented as CFU/mL | | | | | | |

Table 14. Challenge test for FF180432 - BAK (0.02%) and EDTA (0.5%)

| Microorganism | Elapsed time (days) | | | | | Criteria |
|---|---|---|---|---|---|---|
| | 0 | 2 | 7 | 14 | 28 | |
| *P. aeruginosa* | 2.0 x $10^6$ | <10 | <10 | <10 | <10 | **A** |
| *S. aureus* | 1.3 x $10^6$ | 6.5 x $10^3$ | <$10^2$ | <$10^2$ | <$10^2$ | **A** |
| *C. albicans* | 6.1 x $10^5$ | 9.9 x $10^4$ | 1.6 x $10^3$ | 1.0 x $10^2$ | <10 | **A** |
| *A. brasiliensis* | 1.4 x $10^6$ | 6.5 x $10^5$ | 1.1 x $10^6$ | 2.2 x $10^5$ | 2.0 x $10^5$ | **No A or B** |
| *B. cepacia* | 1.0 x $10^6$ | 1.4 x $10^5$ | 1.2 x $10^4$ | 2.9 x $10^2$ | <10 | **B** |
| Results represented as CFU/mL | | | | | | |

The results show that the combination of BAK with chlorhexidine, chlorocresol, methylparaben + propyl-paraben or EDTA does not allow adequate antimicrobial activity of the formulation (Tables 9-14). Further tests (not shown) also resulted in negative results when the preservative is BAK in combination with EDTA (0.5%) and Potassium sorbate (0.008%)

Conversely, the combinations of BAK with either of Cetrimide, Phenylethanol, Thimerosal or Benzyl alcohol comply with the criteria of Ph. Eur. for antimicrobial activity of nasal formulations (Tables 2-8).

**Example 4 - Antimicrobial efficacy of BAK in formulations comprising bilastine or mometasone**

The present example shows the antimicrobial efficacy of BAK when used alone in formulations comprising bilastine or mometasone.

The formulations comprising bilastine were prepared following Process I described above, whereas the formulations comprising mometasone (mometasone Furoate monohydrate) were prepared following an adapted form of Process II described above (by omitting the steps related to bilastine or cyclodextrin).

In the present example, the formulations comprising mometasone only comprise 0.517 mg/mL of Mometasone furoate monohydrate, 2 mg/mL of citric acid monohydrate, 21 mg/mL of glycerin and 0.1 mg/mL of Polysorbate 80 (tween 80) and a pH of 4.6 (Trisodium citrate dihydrate). The table below shows the further excipients in the composition of the formulations of the present example that comprise mometasone only:

| Formulation | Microcrystalline cellulose (MCC) and sodium carboxymethylcelulose (Na-CMC) | benzalkonium chloride |
|---|---|---|
| FF180228 | Avicel RC591 (20 mg/mL) | 0.10 mg/mL |
| FF180229 | | 0.15 mg/mL |
| FF180230 | | 0.20 mg/mL |

(continued)

| Formulation | Microcrystalline cellulose (MCC) and sodium carboxymethylcelulose (Na-CMC) | benzalkonium chloride |
|---|---|---|
| FF180232* | Vivapur 811P (18 mg/mL)) | 0.20 mg/mL |
| FF180233 | | 0.10 mg/mL |
| FF180234 | | 0.15 mg/mL |
| FF180235 | | 0.20 mg/mL |
| *Comparative formulation corresponding to the commercial pharmaceutical composition known as Nasonex®. | | |

In the present example, the formulations comprising bilastine only comprise 8 mg/mL of bilastine, 3 mg/mL of citric acid monohydrate, 21 mg/mL of glycerin and a pH of 4.6 (Trisodium citrate dihydrate). The table below shows the further excipients in the composition of the formulations of the present example that comprise bilastine only:

| Formulation | Microcrystalline cellulose and (MCC) and sodium carboxymethylcellulose (Na-CMC) | benzalkonium chloride |
|---|---|---|
| FF180263 | Avicel RC591 (18 mg/mL) | 0.10 mg/mL |
| FF180264 | | 0.15 mg/mL |
| FF180265 | | 0.20 mg/mL |

The formulations were submitted to the challenge test described in Example 2 above and results showed that while those formulations comprising mometasone only had adequate antimicrobial activity (complying with criteria A of Ph. Eur.), those with bilastine only did not.

**Example 5 - Antimicrobial efficacy in formulations comprising increasing amounts of bilastine and a combination of BAK with benzyl alcohol**

The present example shows the antimicrobial efficacy of BAK when used in combination with benzyl alcohol in formulations comprising bilastine.

The challenged formulations were as described above in example 1, except for those components shown in the table below. The challenge test was as described in example 2.

| Component | Formulation | | |
|---|---|---|---|
| | **181106-1** | **181106-2** | **181106-3** |
| Bilastine | **2** | 5 | 8 |
| benzalkonium chloride (BAK) | 0.2 | 0.2 | 0.2 |
| Hydroxypropyl-β-cyclodextrin | 6.25 | 15.63 | 25 |
| Citric acid monohydrate | 2 | 2.5 | 3 |
| Benzyl alcohol | 5 | 5 | 5 |

Increasing the bilastine concentration does not affect the antimicrobial efficacy of the combined preservatives, and the formulations shown in the present example comply with criteria A (Ph. Eur.) for the six tested microorganisms: *P. aeruginosa, S. aureus, C. albicans, A. brasiliens, E. coli, B. cepacia,* for at the least 28 days required by Ph. Eur.

This example is proof that the combination of BAK with benzyl alcohol is an adequate combination that shows antimicrobial activity in a pharmaceutical composition comprising bilastine.

**Example 6 - Homogeneity de *visu* assessment using HPBCD as a solubilising agent**

The formulation of this example was prepared as explained above in section "Process II - Bilastine and mometasone nasal formulation". The table below shows the content of the components in the final formulation of this example

of the invention:

| Component | mg/mL |
|---|---|
| Bilastine | 4 |
| Mometasone Furoate monohydrate | 0.517 |
| Citric acid monohydrate | 2 |
| Trisodium citrate dihydrate | q.s pH 4.6 |
| Anhydrous glycerine | 21 |
| Polysorbate 80 (Tween 80) | 0.1 |
| Microcrystalline cellulose and sodium carboxymethylcellulose (Vivapur MCG) | 18 |
| benzalkonium chloride | 0.2 |
| Hydroxypropyl-β-cyclodextrin | 25 |
| Purified water | q.s. 1 mL |
| *0.517 mg/mL of Mometasone furoate monohydrate are equivalent to 0.5 mg/mL of Mometasone furoate in anhydrous form. | |

Two further aqueous pharmaceutical compositions of the invention were prepared having the same compositions as in the above composition but changing the bilastine content to 2 mg/mL and to 8 mg/mL.
pH determination: 4.6

Homogeneity *de visu* assessment: the formulation was homogeneous.

**Example 7 - comparative. Homogeneity de *visu* assessment using alternative solubilizer agents of Bilastine and Mometasone**

Five formulations wherein the cyclodextrin was replaced with alternative solubilizer agents of Bilastine and mometasone were prepared and their homogeneity de *visu* assessment was made. The formulations of the present example were prepared in a similar way as explained above (Process II - Bilastine and mometasone nasal formulation) but this time cyclodextrin was replaced with Labrasol® (8 g.), Brij® 35 (8 g.) (Fagron), Myrj® 40 (8 g.) (Fagron), Tween® 80 (8 g.) (Basf) or, Poloxamer 188 (10 g.) (Basf) respectively, due to their surfactant properties. In the formulations of the present example, Span 80 acts as an antifoam agent.

**7.1 Homogeneity de *visu* assessment using Labrasol®.**

Table below shows the exact content of the components in the final formulation of the present example using Labrasol® as a solubilizer agent of bilastine and mometasone:

| Component | % |
|---|---|
| Bilastine | 0.36 |
| Mometasone Furoate | 0.05 |
| Vivapur® MCG | 1.25 |
| Labrasol® | 3.6 |
| Sodium citrate hemihydrate | q.s pH 4.4 |
| Citric acid anhydrous | 0.18 |
| Deionized water | q.s 100 g |
| Tween 80 | 0.0075 |
| Glycerol | 1.8 |
| benzalkonium chloride | 0.015 |

(continued)

| Component | % |
|---|---|
| Span 80 | 0.30 |

Homogeneity *de visu* assessment: there was a phase separation, thus the formulation was not homogeneous.

### 7.2 Homogeneity de *visu* assessment using Myrj® 40.

Table below shows the exact content of the components in the final formulation of the present example using Myrj® 40 as a solubilizer agent of bilastine and mometasone:

| Component | % |
|---|---|
| Bilastine | 0.36 |
| Mometasone Furoate | 0.05 |
| Vivapur® MCG | 1.25 |
| Myrj 40 | 3.6 |
| Sodium citrate hemihydrate | q.s pH 4.4 |
| Citric acid anhydrous | 0.18 |
| Deionized water | q.s 100 g |
| Tween 80 | 0.0075 |
| Glycerol | 1.8 |
| benzalkonium chloride | 0.015 |
| Span 80 | 0.30 |

Homogeneity *de visu* assessment: a coagulated mass was formed at the bottom of the container, thus the formulation was not homogeneous.

### 7.3 Homogeneity de *visu* assessment using Brij® 35.

Table below shows the exact content of the components in the final formulation of the present example using Brij® 35 as a solubilizer agent of bilastine and mometasone:

| Component | % |
|---|---|
| Bilastine | 0.36 |
| Mometasone Furoate | 0.05 |
| Vivapur® MCG | 1.25 |
| Brij 35 | 3.6 |
| Sodium citrate hemihydrate | q.s pH 4.4 |
| Citric acid anhydrous | 0.18 |
| Deionized water | q.s 100 g |
| Tween 80 | 0.0075 |
| Glycerol | 1.8 |
| benzalkonium chloride | 0.015 |
| Span 80 | 0.30 |

Homogeneity *de visu* assessment: there was a significant amount of foam, thus the formulation was not homogeneous.

**7.4 Homogeneity de *visu* assessment using Tween 80.**

Table below shows the exact content of the components in the final formulation of the present example using Tween 80 as a solubilizer agent of bilastine and mometasone:

| Component | % |
|---|---|
| Bilastine | 0.36 |
| Mometasone Furoate | 0.05 |
| Vivapur® MCG | 1.25 |
| Tween 80 | 0.0075 |
| Sodium citrate hemihydrate | q.s pH 4.4 |
| Citric acid anhydrous | 0.18 |
| Deionized water | q.s 100 g |
| Glycerol | 1.8 |
| benzalkonium chloride | 0.015 |
| Span 80 | 0.30 |

Homogeneity *de visu* assessment: there was a phase separation, thus the formulation was not homogeneous.

**7.5 Homogeneity de *visu* assessment using Poloxamer 188.**

Table below shows the exact content of the components in the final formulation of the present example using Poloxamer 188 as a solubilizer agent of bilastine and mometasone:

| Component | % |
|---|---|
| Bilastine | 0.36 |
| Mometasone Furoate | 0.05 |
| Vivapur® MCG | 1.25 |
| Poloxamer 188 | 4.5 |
| Sodium citrate hemihydrate | q.s pH 4.4 |
| Citric acid anhydrous | 0.18 |
| Deionized water | q.s 100 g |
| Tween 80 | 0.0075 |
| Glycerol | 1.8 |
| benzalkonium chloride | 0.015 |
| Span 80 | 0.30 |

Homogeneity *de visu* assessment: there was a phase separation, thus the formulation was not homogeneous.

**Example 8. Solubility of Bilastine and Mometasone furoate monohydrate formulations using amounts of HPBCD from 12.5 to 100 mg/mL at pH values of 4.3, 4.6 and 4.9**

Six different formulations (with mometasone furoate monohydrate and the rest of excipients as defined in example 7) were prepared (see table below). The six formulations had amounts of HPBCD from 12.5 to 100 mg/mL.

Then, in each of them an excessive amount of bilastine was added and the maximum solubility was tested. Furthermore, the percentage of the mometasone furoate dissolved was also tested. All the measurements were conducted at time 0.

| Formulation (HPBCD concentration) | pH | Solubility Mometasone (mg/mL) | % Mometasone dissolved in the drug product | Solubility Bilastine (mg/mL) |
|---|---|---|---|---|
| **1** (12.5 mg/mL) | 4.3 | 0.003 | 0.6 % | 4.3 |
| | | 0.003 | 0.6 % | 4.3 |
| | 4.6 | 0.003 | 0.6 % | 4.5 |
| | | 0.003 | 0.6 % | 4.6 |
| | 4.9 | 0.003 | 0.6 % | 4.8 |
| | | 0.003 | 0.6 % | 4.8 |
| **2** (18.75 mg/mL) | 4.3 | 0.005 | 1.0 % | 4.3 |
| | | 0.005 | 1.0 % | 4.3 |
| | 4.6 | 0.005 | 1.0 % | 4.6 |
| | | 0.005 | 1.0 % | 4.6 |
| | 4.9 | 0.005 | 1.0 % | 4.8 |
| | | 0.005 | 1.0 % | 4.9 |
| **3** (25 mg/mL) | 4.3 | 0.006 | 1.2 % | 12.0 |
| | | 0.006 | 1.2 % | 11.9 |
| | 4.6 | 0.007 | 1.4 % | 9.6 |
| | | 0.006 | 1.2 % | 9.8 |
| | 4.9 | 0.006 | 1.2 % | 8.0 |
| | | 0.007 | 1.4 % | 8.1 |
| **4** (50 mg/mL) | 4.3 | 0.016 | 3.2 % | 16.1 |
| | | 0.013 | 2.6 % | 16.0 |
| | 4.6 | 0.015 | 3.0 % | 13.1 |
| | | 0.016 | 3.2 % | 13.6 |
| | 4.9 | 0.017 | 3.4 % | 12.4 |
| | | 0.015 | 3.0 % | 6.2(*) |
| **5** (75 mg/mL) | 4.3 | 0.019 | 3.8 % | 19.4 |
| | | 0.020 | 4.0 % | 19.7 |
| | 4.6 | 0.021 | 4.2 % | 17.2 |
| | | 0.020 | 4.0 % | 17.4 |
| | 4.9 | 0.023 | 4.6 % | 15.1 |
| | | 0.022 | 4.4 % | 15.2 |

(continued)

| Formulation (HPBCD concentration) | pH | Solubility Mometasone (mg/mL) | % Mometasone dissolved in the drug product | Solubility Bilastine (mg/mL) |
|---|---|---|---|---|
| **6** (100.25 mg/mL) | 4.3 | 0.030 | 6.0 % | 22.9 |
| | | 0.029 | 5.8 % | 20.9 |
| | 4.6 | 0.032 | 6.4 % | 13.7(*) |
| | | 0.031 | 6.2 % | 20.8 |
| | 4.9 | 0.032 | 6.4 % | 18.2 |
| | | 0.032 | 6.4 % | 18.1 |
| (*) A difference between the grams needed to reach saturation in the sample in respect of its corresponding replicate was observable. | | | | |

The results are represented in Figure 1A and 1B. The results show that the desired solubility for the bilastine dosage of 8 mg/mL in the tested pH range of 4.3 - 4.9 is reached when using at least 25 mg/mL of HPBCD at time 0. The results also show that when increasing the content of HPBCD in the tested pH range of 4.3 - 4.9, the content of solubilised steroid is also increased (see table above and Figure 1B).

**Example 9 - comparative. Solubility of Bilastine and Mometasone furoate monohydrate formulations using amounts of Kolliphor RH40 from 0 to 50 mg/mL at pH values of 3.7, 4.0, 4.3 and 4.6**

Kolliphor RH40 (Macrogolglycerol hydroxystearate), a non-ionic oil-in-water solubilizing and emulsifying agent, derived from hydrogenated castor oil and ethylene oxide, was evaluated as solubilizer agent of bilastine and mometasone.

To this end, aqueous formulations of Kolliphor RH40 at concentrations 0, 5, 15, 25 and 50 mg/mL and at pH values of 3.7, 4.0, 4.3 and 4.6 were prepared.

To each of the solubilizer formulations, bilastine was added until saturation was observed in order to determine the maximum concentration of bilastine.

Additionally, 0.5 mg/mL of mometasone furoate monohydrate was added to each formulation. The influence of Kolliphor RH40 in bilastine solubility is represented in Figures 3A and 3B. It is shown that, at pH values above 4.3, Kolliphor RH40 at a concentration of 50 mg/mL is insufficient in solubilizing 8 mg/mL of Bilastine (Figure 3A). In addition, at the concentration of 50 mg/mL and pH of 4.3, Kolliphor RH40 solubilizes more than 10% of the added mometasone furoate monohydrate (Figure 3B).

The results demonstrate that Kolliphor RH40 is an inadequate solubilizer in the context of the present invention since it does not properly solubilize the desired amount of bilastine while simultaneously solubilizes a significant amount of mometasone furoate monohydrate.

**Example 10 - comparative. Stability using Kolliphor RH40**

A comparative formulation wherein the cyclodextrin is replaced with Kolliphor RH40 as solubilizer agent was prepared.

The formulation of the present example was prepared in a similar way as explained above but this time cyclodextrin was replaced with Macrogolglycerol hydroxystearate (Kolliphor RH40) and the quantities of the components was slightly adapted. The table below shows the exact content of the components in the final formulation of the present example:

| Component | mg/mL |
|---|---|
| Bilastine | 4 |
| Mometasone Furoate monohydrate | 0.517 |
| Citric acid monohydrate | 2.19 |
| Trisodium citrate dihydrate | q.s pH 5 |
| Anhydrous glycerine | 20 |

(continued)

| Component | mg/mL |
|---|---|
| Polysorbate 80 (Tween 80) | 0.1 |
| Microcrystalline cellulose and sodium carboxymethylcellulose (Vivapur MCG) | 13.1 |
| benzalkonium chloride | 0.3 |
| Macrogolglycerol hydroxystearate (Kolliphor RH40) | 50 |
| Sorbitan Monooleate (Span 80) | 6 |
| Purified water | q.s. 1 mL |
| *0.517 mg/mL of Mometasone furoate monohydrate are equivalent to 0.5 mg/mL of Mometasone furoate in anhydrous form. | |

In the formulation of the present example, sorbitan monooleate acts as an antifoam agent.

A batch manufactured according to the table above was prepared, packaged and placed in stability chambers wherein the conditions were 25 °C / 40% RH and 40 °C / 25% RH for the duration of 3 months.

| Total degradation products wt% | T0 | 25 °C 40% RH | 40 °C 25% RH |
|---|---|---|---|
| Mometasone Furoate monohydrate | 0.15% | 0.31% | 0.33% |

The results show that the level of mometasone total degradation products is over pharmaceutical acceptable levels. Moreover, the content of mometasone total degradation products increases over time, since its value is doubled from time 0 to 3 months at both stability conditions, 25 °C / 40% RH and 40 °C / 25% RH. The stability of the formula with Kolliphor RH40 as solubilizer agent is considered unsatisfactory. Thus it is concluded that Kolliphor RH40 is not adequate for solubilizing bilastine because not only it does not solubilize enough bilastine (see example above) but it has the unwanted side effect of leading to the degradation of mometasone over pharmaceutical acceptable levels.

**Example 11 - Stability using cyclodextrin**

Three different Bilastine formulas with 2, 4 and 8 mg/mL were prepared in a similar way as explained above and placed into stability chambers. The table below shows the exact content of the components in the three formulas of the present example:

| Component | mg/mL | | |
|---|---|---|---|
| Bilastine | 2 | 4 | 8 |
| Mometasone Furoate monohydrate | 0.517 (1) | 0.517 (1) | 0.517 (1) |
| Citric acid monohydrate | 2.0 | 2.0 | 2.0 |
| HPBCD | 25 | 25 | 25 |
| Anhydrous glycerine | 21 | 21 | 21 |
| Polysorbate 80 (Tween 80) | 0.1 | 0.1 | 0.1 |
| Microcrystalline cellulose and sodium carboxymethylcellulose (Vivapur MCG) | 18 | 18 | 18 |
| benzalkonium chloride | 0.2 | 0.2 | 0.2 |
| Trisodium citrate dihydrate | 1.65 q.s pH 4.6 | 1.65 q.s pH 4.6 | 1.65 q.s pH 4.6 |
| Purified water | q.s 1 mL | q.s 1 mL | q.s 1 mL |
| (1) 0.517 mg/mL of Mometasone furoate monohydrate are equivalent to 0.5 mg/mL of Mometasone in its anhydrous form. | | | |

The three formulas (bilastine at a concentration of 2, 4 and 8 mg/mL) were placed in stability chambers wherein the conditions were 25 °C / 40% RH and 40 °C / 25% RH for the duration of 6 or 12 months.

Bilastine 2 mg/mL

| **Tolal degradation products wt%** | **T0** | **40 °C 25% RH 3 months** | **40 °C 25% RH 6 months** | **25 °C 40% RH 9 months** | **25 °C 40% RH 12 months** |
|---|---|---|---|---|---|
| Mometasone Furoate | 0.05% | 0.15% | 0.20% | 0.05% | 0.05% |

Bilastine 4 mg/mL

| **Total degradation products wt%** | **T0** | **40 °C 25% RH 3 Months** | **40 °C 25% RH 6 months** | **25 °C 40% RH 9 months** | **25 °C 40% RH 12 months** |
|---|---|---|---|---|---|
| Mometasone Furoate | 0.05% | 0.14% | 0.18% | 0.05% | 0.05% |

Bilastine 8 mg/mL

| **Total degradation products wt%** | **T0** | **40 °C 25% RH 3 Months** | **40 °C 25% RH 6 months** | **25 °C 40% RH 9 months** | **25 °C 40% RH 12 months** |
|---|---|---|---|---|---|
| Mometasone Furoate | 0.05% | 0.14% | 0.17% | 0.05% | 0.05% |

As can be seen in the tables above, a slightly increase in the level of mometasone total degradation products is found after 6 months at 40 °C/ 25% RH. This increase is almost the same for the three formulas of the example. This increase is not noticed after 12 months at 25 °C/ 40% RH. Thus, the stability of the three formulas is considered satisfactory and pharmaceutically acceptable.

These results, when compared to those obtained in comparative example 9 show that contrary to Kolliphor RH40, the cyclodextrin HPBCD is sufficient to solubilize 8 mg/mL of bilastine without the unwanted side effects of degradation of mometasone over pharmaceutical acceptable levels.

**Example 12 - Mometasone impurities**

This example shows the data produced by the inventors upon the surprising discovery that there are mometasone impurities originating from the presence of HPBCD as solubilizer.

The percentage of Mometasone was calculated using the following formula:

$$\%\, Mometasone = \frac{Am \times Pstd \times Dm \times F \times d}{Astd \times Dstd \times Pm \times T} \times 100$$

Where:

Am: Area of Mometasone peak in sample solution.

Astd: Mean of peak areas of Mometasone (n=5) in STD1 Solution A

Pstd: Weight of Mometasone in the standard solution (mg)

Pm: Weight of sample (g)

Dstd: Dilution of the Mometasone in the standard solution (mL)

Dm: Dilution of sample solution (mL)

F: Potency of the Mometasone working or reference standard (amount per one)

T: Theoretic quantity of Mometasone in suspension (mg/mL) (0.517mg/mL)

d: Density of sample (g/mL)

The percentage of Mometasone degradation products was calculated using the following formula:

$$\% \, Impurity = \frac{Am \times Pstd \times Dm \times F \times d}{Astd \times Dstd \times Pm \times T \times RRF} \times 100$$

Where:

Am: Area of Mometasone peak in sample solution.

Astd: Mean of peak areas of Mometasone (n=5) in STD1 Solution B

Pstd: Weight of Mometasone in the standard solution (mg)

Pm: Weight of sample (g)

Dstd: Dilution of the Mometasone in the standard solution (mL)

Dm: Dilution of sample solution (mL)

F: Potency of the Mometasone working or reference standard (amount per one)

T: Theoretic quantity of Mometasone in suspension (mg/mL) (0.517mg/mL)

d: Density of sample (g/mL)

RRF: Relative Response Factor of Mometasone degradation product (considered as 1).

To evaluate the influence that the cyclodextrin HPBCD had on the impurities of mometasone, formulations similar to the one described in example 6 (see table therein) were studied at pH 4.6 for increasing cyclodextrin concentrations of from 25 to 95 mg/mL. The formulations were submitted to forced stability conditions at 50 °C for a 3-month period. The results were obtained from HPLC measurements.

| HPBCD concentration | Dissolved mometasone (%) | Mometasone impurities (%) |
|---|---|---|
| 25 mg/mL | 0.7 | 0.2 |
| 50 mg/mL | 3.0 | 0.7 |
| 65 mg/mL | 5.2 | 1.0 |
| 85 mg/mL | 7.2 | 1.3 |
| 95 mg/mL | 10.5 | 1.9 |

The plot of mometasone impurities against solubilized mometasone reveals that there is a direct linear relation between these two parameters, as shown in Figure 4A.

The plot of mometasone impurities against cyclodextrin concentration is represented in Figure 4B. A steady increase in the percentage of mometasone impurities is observed with the increase of HPBCD concentration, in the interval 25-85 mg/mL of cyclodextrin. When the cyclodextrin concentration is increased to 95 mg/mL, the trend is no longer linear and a significant increase in the mometasone impurities is observed.

## Claims

1. An aqueous pharmaceutical composition comprising:

    a) bilastine or a pharmaceutically acceptable salt or solvate thereof,
    b) benzalkonium chloride, in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt;
    c) a suspending agent,
    d) 2-hydroxypropyl-β-cyclodextrin;

    wherein the pH of the aqueous pharmaceutical composition is between 3.5 and 5.5.

2. The aqueous pharmaceutical composition according to claim 1, further comprising e) mometasone or a pharmaceutically acceptable derivative thereof selected from an ester, ether and ketonide derivative, and wherein the content of 2-hydroxypropyl-β-cyclodextrin is less than 8.5 wt%.

3. The aqueous pharmaceutical composition according to any one of claims 1 or 2, wherein the content of bilastine or a pharmaceutically acceptable salt or solvate thereof is comprised between 0.2 wt% and 0.8 wt%.

4. The aqueous pharmaceutical composition according to any one of claims 1 to 3, wherein the total content of preservative is comprised between 0.010 and 2 wt%, preferably between 0.010 and 0.6 wt%.

5. The aqueous pharmaceutical composition according to any one of claims 1 to 4, wherein the content of 2-hydroxypropyl-β-cyclodextrin is comprised between 1 and 5 wt%.

6. The aqueous pharmaceutical composition according to any one of claims 1 to 5, wherein the suspending agent is selected from cellulose and/or cellulose derivatives selected from cellulose ether derivatives wherein the hydroxyl groups of cellulose have been partially or fully substituted to provide cellulose ethers.

7. A process for preparing an aqueous pharmaceutical composition according to any one of claims 1 to 6 comprising:

    a) preparing an aqueous solution comprising

        a1. 2-hydroxypropyl-β-cyclodextrin,
        a2. benzalkonium chloride in combination with at least one further preservative selected from the group comprising phenylethyl alcohol, benzyl alcohol, sodium benzoate, chlorbutanol, phenoxyethanol, cetrimide and 2-(ethylmercuriomercapto)benzoic acid sodium salt,
        a3. bilastine or a pharmaceutically acceptable salt or solvate thereof,
        a4. a buffer agent to obtain an aqueous solution having a pH of between 3.5 and 5.5,

    b) preparing an aqueous suspension of a suspending agent,
    c) preparing a mixture by adding the aqueous solution of step a) to the aqueous suspension of step b) and,
    d) homogenising the mixture of the preceding step under stirring, optionally adding a further buffer to reach a pH of between 3.5 and 5.5.

8. The process according to claim 7, further comprising the step of:
    b1) preparing a dispersion of mometasone, or a pharmaceutically acceptable derivative thereof selected from an ester, ether and ketonide derivative with a surfactant in purified water,
    wherein the dispersion of step b1) is added to the mixture of step c), before the homogenizing step d), and wherein the content of 2-hydroxypropyl-3-cyclodextrin in step a) is less than 8.5 wt%.

9. The process according to any one of claims 7 or 8, wherein the aqueous solution of 2-hydroxypropyl-β-cyclodextrin is an aqueous solution wherein the content of 2-hydroxypropyl-β-cyclodextrin is comprised between 1 and 5 wt%.

10. An aqueous pharmaceutical composition according to any one of claims 1 to 6, for use as a medicament.

11. An aqueous pharmaceutical composition according to any one of claims 1 to 6, for use in the treatment and/or

prevention of a disorder or disease susceptible to amelioration by antagonism of $H_1$ histamine receptor and/or of a corticosteroid-responsive disease.

**12.** The aqueous pharmaceutical composition for use according to claim 11, wherein the disorder or disease susceptible to amelioration by antagonism of H1 histamine receptor is an allergic disorder or disease selected from rhinitis, conjunctivitis and rhinoconjunctivitis.

**13.** The aqueous pharmaceutical composition for use according to claim 11, wherein the corticosteroid-responsive disease is selected from asthma, allergic and non-allergic rhinitis, non-malignant proliferative and inflammatory diseases.

**14.** The aqueous pharmaceutical composition for use according to any one of claims 10 to 13 wherein the aqueous pharmaceutical composition is intranasally administered.

**15.** A nasal spray device comprising the aqueous pharmaceutical composition according to any one of claims 1 to 6.

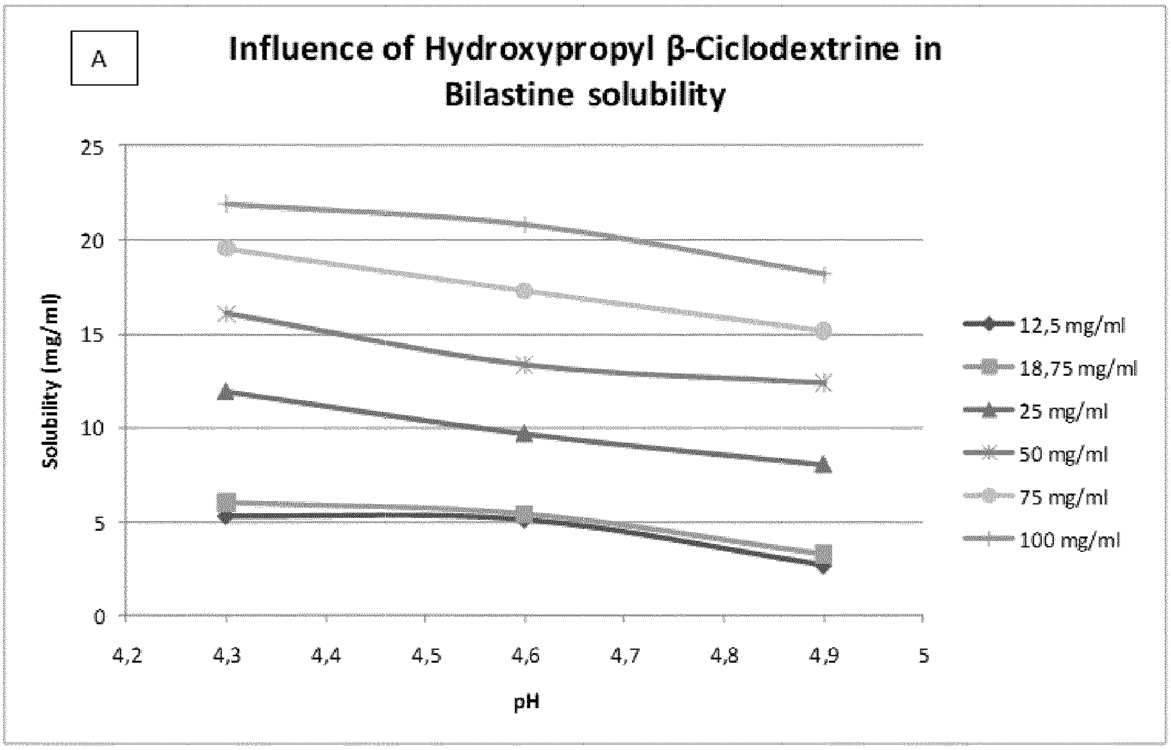
A
Influence of Hydroxypropyl β-Ciclodextrine in Bilastine solubility
Solubility (mg/ml)
25
20
15
10
5
0
4,2
4,3
4,4
4,5
4,6
4,7
4,8
4,9
5
pH
12,5 mg/ml
18,75 mg/ml
25 mg/ml
50 mg/ml
75 mg/ml
100 mg/ml

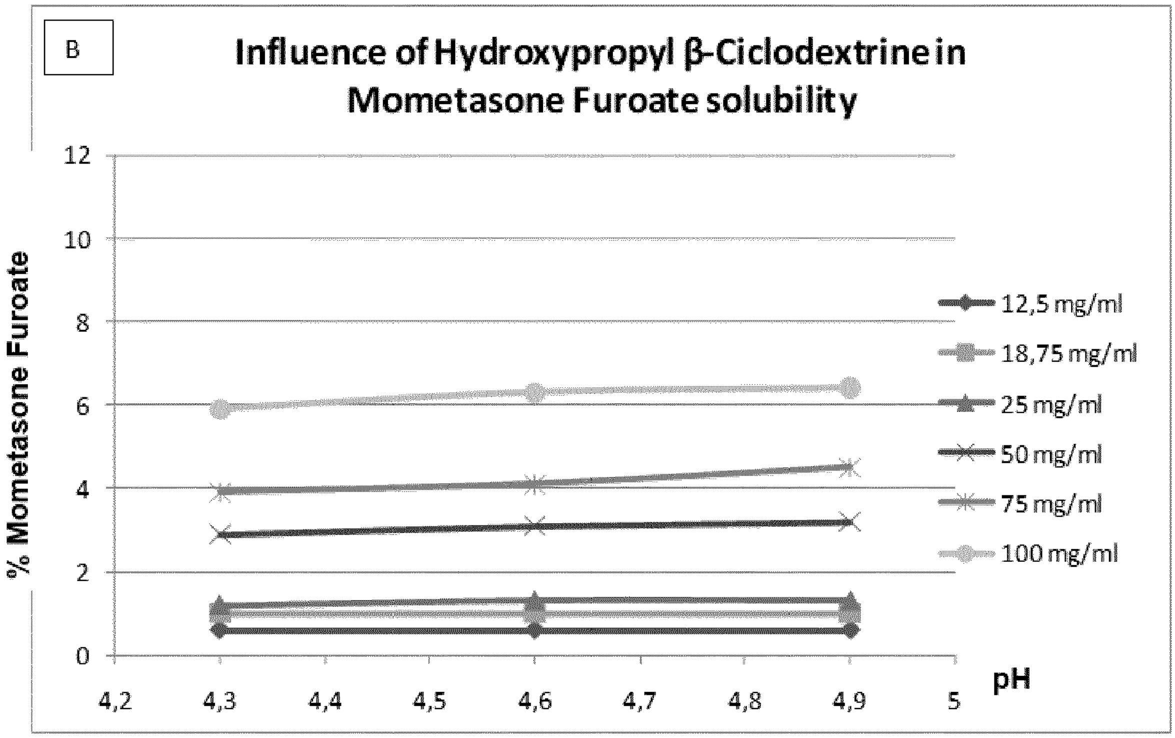
B
Influence of Hydroxypropyl β-Ciclodextrine in Mometasone Furoate solubility
% Mometasone Furoate
12
10
8
6
4
2
0
4,2
4,3
4,4
4,5
4,6
4,7
4,8
4,9
5
pH
12,5 mg/ml
18,75 mg/ml
25 mg/ml
50 mg/ml
75 mg/ml
100 mg/ml

Figure 1

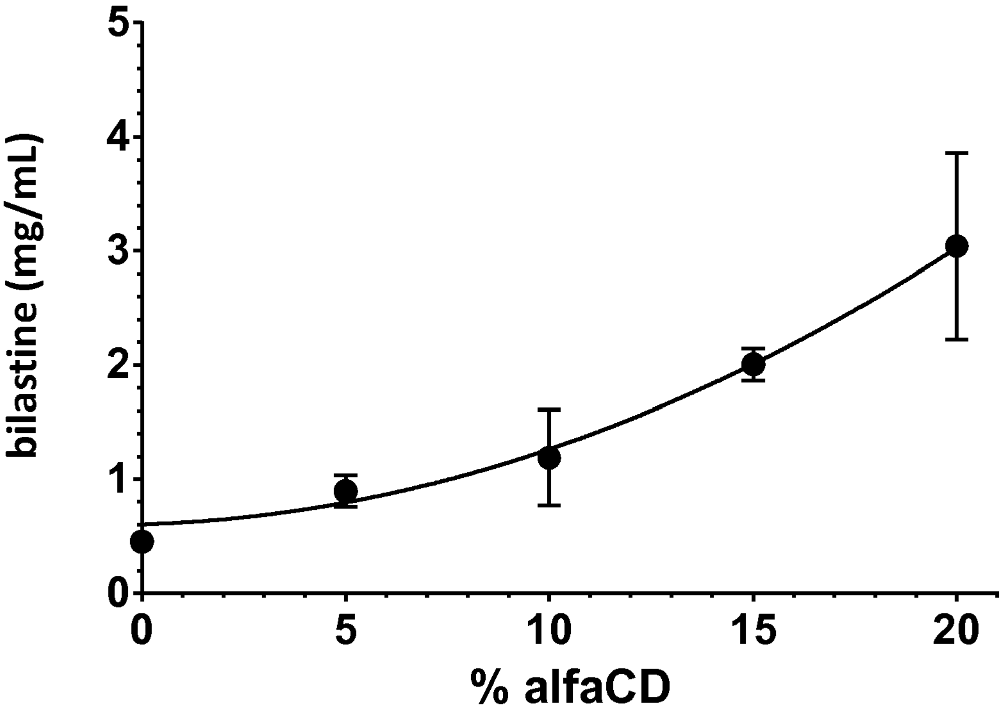


Figure 2

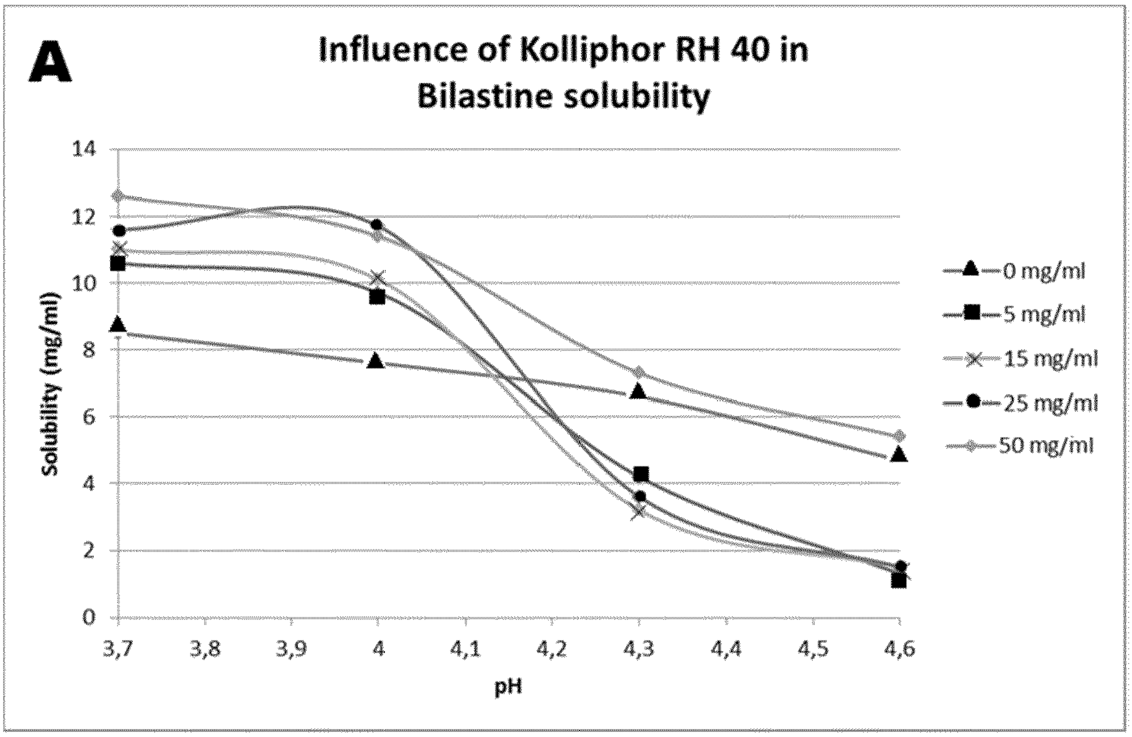
A
Influence of Kolliphor RH 40 in
Bilastine solubility
Solubility (mg/ml)
14
12
10
8
6
4
2
0
3,7
3,8
3,9
4
4,1
4,2
4,3
4,4
4,5
4,6
pH
0 mg/ml
5 mg/ml
15 mg/ml
25 mg/ml
50 mg/ml

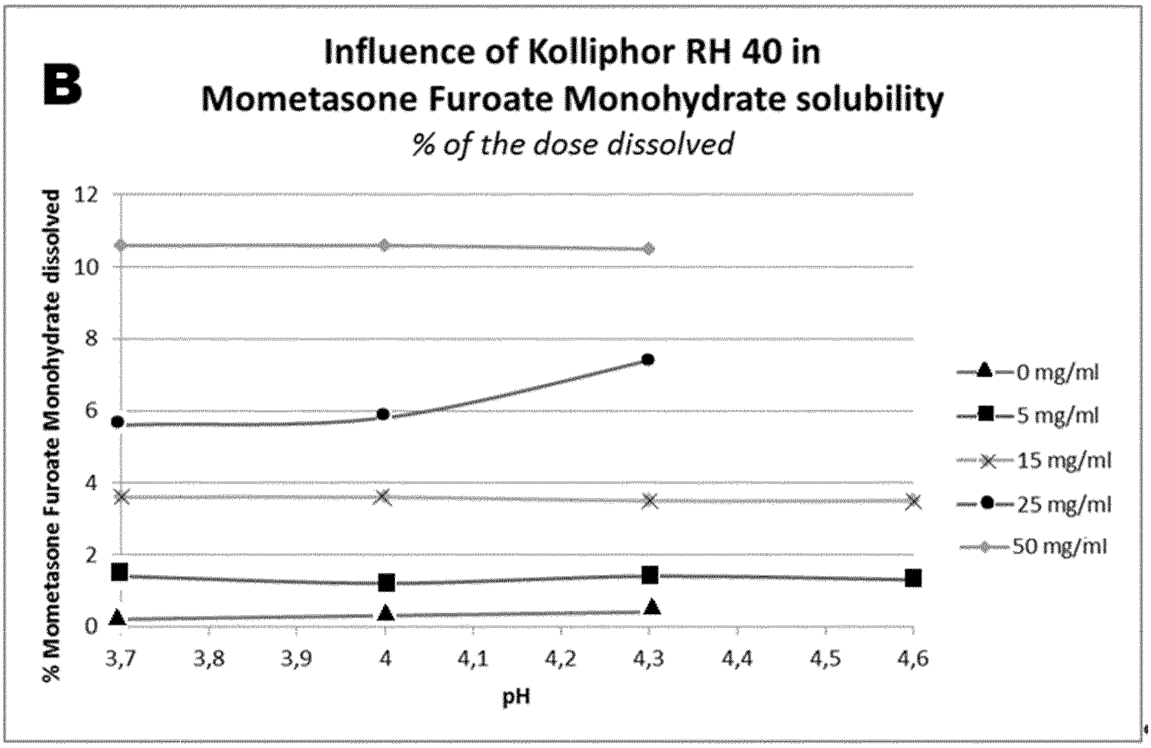
B
Influence of Kolliphor RH 40 in
Mometasone Furoate Monohydrate solubility
% of the dose dissolved
% Mometasone Furoate Monohydrate dissolved
12
10
8
6
4
2
0
3,7
3,8
3,9
4
4,1
4,2
4,3
4,4
4,5
4,6
pH
0 mg/ml
5 mg/ml
15 mg/ml
25 mg/ml
50 mg/ml

Figure 3

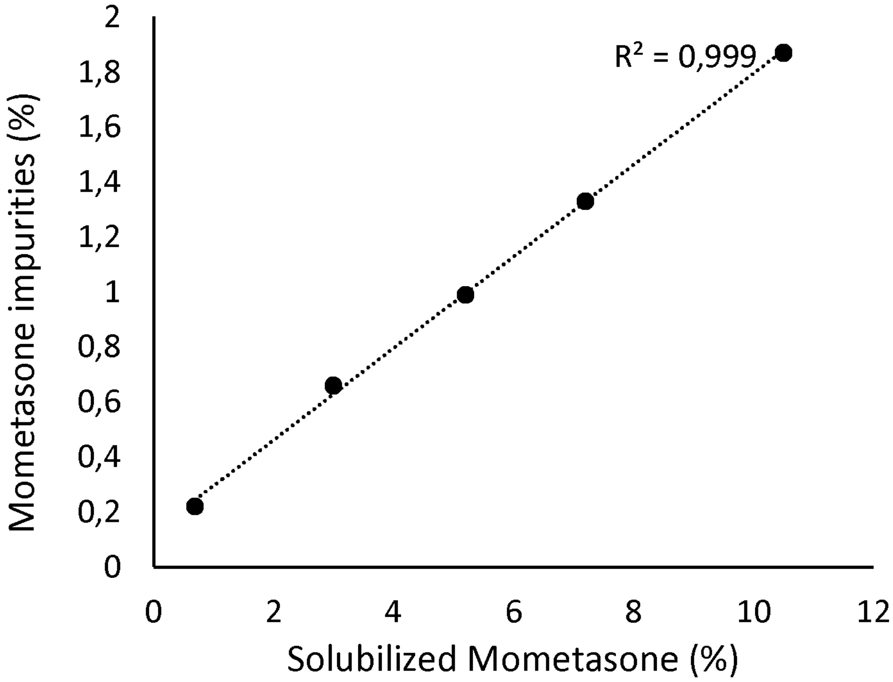


**Figure 4A**

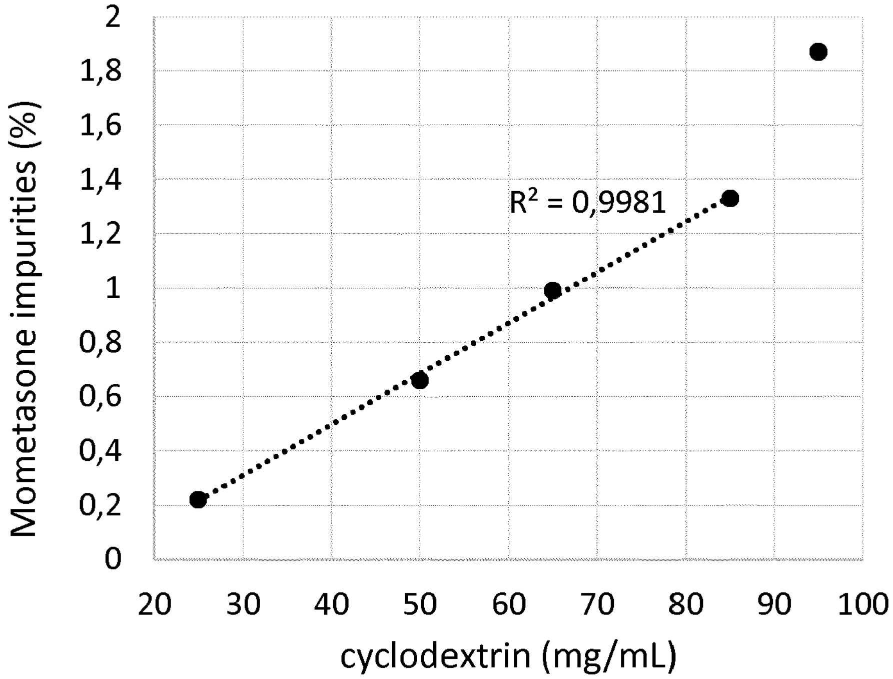


**Figure 4B**

Europäisches Patentamt
European Patent Office
Office européen des brevets

# EUROPEAN SEARCH REPORT

Application Number
EP 19 38 2298

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 103 784 462 A (SONG JUNZHI) 14 May 2014 (2014-05-14)<br>* examples 1-7 *<br>* claims 1-10 * | 1-15 | INV.<br>A61K9/00<br>A61K9/08<br>A61K31/454<br>A61K31/58<br>A61K47/10<br>A61K47/12<br>A61K47/18<br>A61K47/24<br>A61K47/38<br>A61K47/40<br>A61K47/69 |
| A | CN 103 736 098 A (YU YUNHONG) 23 April 2014 (2014-04-23)<br>* examples * | 1-15 | |
| A | US 2009/312724 A1 (PIPKIN JAMES D [US] ET AL) 17 December 2009 (2009-12-17)<br>* examples *<br>* claims * | 1-15 | |
| | | | TECHNICAL FIELDS SEARCHED (IPC) |
| | | | A61K |

The present search report has been drawn up for all claims

| Place of search | Date of completion of the search | Examiner |
|---|---|---|
| The Hague | 14 October 2019 | Marchand, Petra |

5

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 38 2298

This annex lists the patent family members relating to the patent documents cited in the above-mentioned European search report. The members are as contained in the European Patent Office EDP file on
The European Patent Office is in no way liable for these particulars which are merely given for the purpose of information.

14-10-2019

| Patent document cited in search report | Publication date | Patent family member(s) | Publication date |
|---|---|---|---|
| CN 103784462 A | 14-05-2014 | NONE | |
| CN 103736098 A | 23-04-2014 | NONE | |
| US 2009312724 A1 | 17-12-2009 | ES 2493641 T3 | 12-09-2014 |
| | | US 2009312724 A1 | 17-12-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0818454 A1
- EP 0580541 A1
- EP 14382576 A
- WO 2005082416 A
- WO 2012094283 A
- WO 2013078500 A
- CN 103784462
- KR 20130030606 A
- KR 20100119632
- US 2006045850 A1
- EP 1894559 A1
- US 2007082870 A1


### Non-patent literature cited in the description


- **SUSAN A. DOLAN et al.** An Outbreak of Burkholderia cepacia Complex Associated with Intrinsically Contaminated Nasal Spray. *Infect Control Hosp Epidemiol,* 2011, vol. 32 (8), 804-810
- **M.B. SMITH ; J. MARCH.** March's Advanced Organic Chemistry. Wiley-Interscience
- Remington, The Science and practice of pharmacy. 2005, 072
- **SIMONS, F. ; ESTELLE R. ; KEITH J. SIMONS.** Histamine and H1-antihistamines: celebrating a century of progress. *Journal of Allergy and Clinical Immunology,* 2011, vol. 128.6, 1139-1150
- **KALPAKLIOGLU, FUSUN ; AYSE BACCIOGLU.** Efficacy and safety of H1-antihistamines: an update. *Anti-Inflammatory & Anti-Allergy Agents in Medicinal Chemistry (Formerly Current Medicinal Chemistry-Anti-Inflammatory and Anti-Allergy Agents),* 2012, vol. 11.3, 230-237